# EUROPEAN PATENT APPLICATION

(11) **EP 4 488 365 A1**
(43) Date of publication of application: **08.01.2025**
(21) Application number: 23763341.7
(22) Date of filing: 22.02.2023
(51) Int. Cl.: C12N 5/077, A61K 35/36, A61P 17/14

(54) **METHOD FOR PRODUCING PROLIFERATED HAIR FOLLICLE MESENCHYMAL CELLS, AND USE THEREOF**

(30) Priority: 01.03.2022 JP 2022031086
(71) Applicant: NATIONAL UNIVERSITY CORPORATION YOKOHAMA NATIONAL UNIVERSITY, Yokohama-shi Kanagawa 240-8501 (JP); KANAGAWA INSTITUTE OF INDUSTRIAL SCIENCE AND TECHNOLOGY, Ebina-shi Kanagawa 243-0435 (JP)
(72) Inventor: FUKUDA, Junji, Yokohama-shi, Kanagawa 240-8501 (JP); KAGEYAMA, Tatsuto, Ebina-shi, Kanagawa 243-0435 (JP); YAN, Lei, Yokohama-shi, Kanagawa 240-8501 (JP)
(74) Representative: Winter, Brandl - Partnerschaft mbB
(86) International application number: PCT/JP2023/006551
(87) International publication number: WO 2023/167082

(57) **Abstract**

Provided are a method of producing hair follicle mesenchymal cells whose number has been expanded and whose hair regeneration abilities have been recovered, and a use thereof. The method of producing expanded hair follicle mesenchymal cells includes a main step of culturing hair follicle mesenchymal cells adhered to a substrate surface from a confluent state without passaging the cells. The main step includes culturing the hair follicle mesenchymal cells to make the cells proliferate further from the confluent state until the proliferated hair follicle mesenchymal cells are stacked to form cell layers including two or more stacked layers in at least part of the substrate surface.

## Description

### Technical Field

The present invention relates to a method of producing expanded hair follicle mesenchymal cells, and a use thereof.

### Background Art

In Non Patent Literature 1, there is a description of a primary culture method including: mounting dermal papillae separated from human hair follicles on a plastic petri dish in a culture solution; and causing dermal papilla cells to migrate from the dermal papillae onto the bottom surface of the dish to culture the cells.

In Non Patent Literature 2, there is a description of the result of an investigation on a culture method for maintaining the functions of human dermal papilla cells in vitro. In Non Patent Literature 2, there are also a description that when dermal papilla cells reached a confluent state in their primary culture, the cells were harvested, and were reseeded to a 60-millimeter dish at a cell density of 20,000 cells/dish, and a description that subculture was further performed thereafter.

In Patent Literature 1, there is a description of a method of culturing dermal papilla cells each maintaining a hair-inducing ability, the method being characterized by including culturing the dermal papilla cells in the presence of an FGF-based material, a BMP-2/BMP-4-based material, and a WNT-based material. In Patent Literature 1, there is also a description that in general, to sufficiently secure the number of the cells, and to appropriately keep the density of the cells to maintain a satisfactory growing ability, the following passage is required: cultured cells are harvested before the inside of a culture tool becomes overcrowded with the cells, and the number of the cells is adjusted, followed by their renewed culture.

In Patent Literature 2, there is a description of a method of isolating hair follicle mesenchymal stem cells, the method including the steps of: (a) preparing vigorous hair; (b) dividing the hair prepared in the step (a); (c) isolating a cup-like substance-adhered hair cup together with a dermal hair papilla; (d) separating the dermal hair papilla from the hair cup; (e) culturing the hair cup obtained in the step (d); and (f) pooling confluent cells. In Patent Literature 2, there is a description that subculture including the following was performed: dermal sheath cup (DSC) cells collected from the hair follicles of a mouse were cultured in a 24-well culture flask; and after having reached a confluent state, the cells were separated with trypsin-EDTA and transferred to a 25-milliliter culture flask.

In Non Patent Literature 3, there is a description that while the two-dimensional culture of dermal papilla cells collected from human hair follicles reduce their in vivo characteristics, when the dermal papilla cells passaged by the two-dimensional culture are cultured by a hanging drop method to form a spheroid, the in vivo characteristics of the dermal papilla cells which form the spheroid recover.

### Citation List

### Patent Literature

[PTL 1] WO 2010-021245 A1
[PTL 2] JP 2005-528916 A

### Non Patent Literature

[NPL 1] A.G. MESSENGER, British Journal of Dermatology (1984) 110, 685-689
[NPL 2] M. Ohyama et al., Journal of Cell Science (2012) 125, 4114-4125
[NPL 3] Claire A. Higgins et al., PNAS (2013) vol. 110, no. 49, 19679-19688

### Summary of Invention

### Technical Problem

The following has heretofore been performed for expanding the number of dermal papilla cells: the dermal papilla cells are two-dimensionally cultured, and their passage is repeated. However, it has been common technical knowledge that the hair regeneration abilities of the two-dimensionally cultured dermal papilla cells largely reduce.

Meanwhile, when dermal papilla cells are three-dimensionally cultured, that is, cultured through the formation of a spheroid from the dermal papilla cells, the hair regeneration abilities of the dermal papilla cells which form the spheroid recover to some extent. However, it has been difficult to expand the number of the dermal papilla cells that have formed the spheroid.

The present invention has been made in view of the above-mentioned problems, and one of the objects of the present invention is to provide hair follicle mesenchymal cells whose number has been expanded and whose hair regeneration abilities have recovered, and a use thereof.

### Solution to Problem

[1] In order to achieve the above-mentioned object, according to one aspect of one embodiment of the present invention, there is provided a method of producing expanded hair follicle mesenchymal cells, including a main step of culturing hair follicle mesenchymal cells adhered to a substrate surface from a confluent state without passaging the cells, wherein the main step includes culturing the hair follicle mesenchymal cells to make the cells proliferate further from the confluent state until the proliferated hair follicle mesenchymal cells are stacked to form cell layers including two or more stacked layers in at least part of the substrate surface. According to the embodiment of the present invention, a method of producing hair follicle mesenchymal cells whose number has been expanded and whose hair regeneration abilities have recovered is provided.
[2] In the method of the above-mentioned item [1], the main step may include culturing the hair follicle mesenchymal cells until a time period of 120 hours or more elapses from the confluent state. [3] In the method of the above-mentioned item [1] or [2], the main step may include culturing the hair follicle mesenchymal cells until a cell density of the hair follicle mesenchymal cells on the substrate surface reaches 7×10⁴ cells/cm² or more. [4] In the method according to any one of the above-mentioned items [1] to [3], the main step may include culturing the hair follicle mesenchymal cells for 50 hours or more in a state in which a cell density of the hair follicle mesenchymal cells on the substrate surface is 7×10⁴ cells/cm² or more. [5] In the method according to any one of the above-mentioned items [1] to [4], the main step may include culturing the hair follicle mesenchymal cells until a cell density of the hair follicle mesenchymal cells on the substrate surface reaches 1.5 or more times as high as that in the confluent state. [6] In the method according to any one of the above-mentioned items [1] to [5], the main step may include culturing the hair follicle mesenchymal cells until an expression amount of each of one or more hair growth-related genes of the hair follicle mesenchymal cells increases to 1.5 or more times as large as that in the confluent state.
[7] The method according to any one of the above-mentioned items [1] to [6] may further include a pre-step to be performed prior to the main step, wherein the pre-step may include culturing the hair follicle mesenchymal cells that have not yet reached the confluent state on the substrate surface to make the cells proliferate until the hair follicle mesenchymal cells reach the confluent state. [8] In the method according to the above-mentioned item [7], the pre-step may include culturing the hair follicle mesenchymal cells at a cell density of 2.5×10⁴ cells/cm² or less to make the cells proliferate until the hair follicle mesenchymal cells reach the confluent state. [9] In the method according to the above-mentioned item [7] or [8], the main step may include culturing the hair follicle mesenchymal cells until a time period of 200 hours or more elapses from a start of the culture of the hair follicle mesenchymal cells in the pre-step. [10] In the method according to any one of the above-mentioned items [7] to [9], the main step may include culturing the hair follicle mesenchymal cells until an expression amount of each of one or more hair growth-related genes of the hair follicle mesenchymal cells increases to 1.1 or more times as large as that at a time point when 24 hours elapses from a start of the culture of the hair follicle mesenchymal cells in the pre-step.
[11] The method according to any one of the above-mentioned items [1] to [10] may further include a harvesting step of detaching the hair follicle mesenchymal cells, which have been obtained through the culture on the substrate surface from the confluent state without the passage in the main step, from the substrate surface, followed by harvesting the detached cells. [12] In the method according to any one of the above-mentioned items [1] to [11], the hair follicle mesenchymal cells may be one or more selected from the group consisting of: dermal papilla cells; and dermal sheath cup cells.
[13] In order to achieve the above-mentioned object, according to another aspect of one embodiment of the present invention, there is provided a method of producing a cell aggregate, including forming a cell aggregate containing expanded hair follicle mesenchymal cells produced by the method of any one of the above-mentioned items [1] to [12]. According to the embodiment of the present invention, a method of producing a cell aggregate containing hair follicle mesenchymal cells whose number has been expanded and whose hair regeneration abilities have recovered is provided.
[14] In order to achieve the above-mentioned object, according to still another aspect of one embodiment of the present invention, there is provided a method of producing a composition for transplantation, including preparing a composition for transplantation containing expanded hair follicle mesenchymal cells produced by the method of any one of the above-mentioned items [1] to [12]. According to the embodiment of the present invention, a method of producing a composition for transplantation containing hair follicle mesenchymal cells whose number has been expanded and whose hair regeneration abilities have recovered is provided.
[15] In order to achieve the above-mentioned object, according to still another aspect of one embodiment of the present invention, there is provided a hair regeneration method, including transplanting, to a living body, expanded hair follicle mesenchymal cells produced by the method of any one of the above-mentioned items [1] to [12]. According to the embodiment of the present invention, a hair regeneration method using hair follicle mesenchymal cells whose number has been expanded and whose hair regeneration abilities have recovered is provided.

### Advantageous Effects of Invention

According to the present invention, the hair follicle mesenchymal cells whose number has been expanded and whose hair regeneration abilities have recovered, and the use thereof, are provided.

### Brief Description of Drawings

FIG. 1 is an explanatory figure showing Giemsa staining images of dermal papilla cells cultured in the example 1 of Example 1.
FIG. 2 is an explanatory figure showing micrographs of dermal papilla cells (spheroid) cultured in the example C1 of Example 1.
FIG. 3 is an explanatory figure showing results of determination of the numbers of the dermal papilla cells cultured in Example 1.
FIG. 4 is an explanatory figure showing the results of determination of the ALP gene expression of the dermal papilla cells cultured in Example 1.
FIG. 5 is an explanatory figure showing the results of observation of cell layers of the dermal papilla cells cultured in the example 1 of Example 1 with a confocal laser microscope.
FIG. 6A is a set of explanatory diagrams showing the results of immunofluorescent double staining of fibronectin and cell nuclei in the example 1 of Example 1.
FIG. 6B is an explanatory figure showing only the immunofluorescent staining images of fibronectin out of the fluorescent double staining images shown in FIG. 6A.
FIG. 6C is an explanatory figure showing the results of the immunofluorescent double staining of type I collagen and the cell nuclei in the example 1 of Example 1.
FIG. 6D is an explanatory figure showing only the immunofluorescent staining images of type I collagen among the fluorescent double staining images shown in FIG. 6C.
FIG. 6E is an explanatory figure showing the results of the immunofluorescent double staining of alkaline phosphatase and the cell nuclei in the example 1 of Example 1.
FIG. 6F is an explanatory figure showing only the immunofluorescent staining images of alkaline phosphatase among the fluorescent double staining images shown in FIG. 6E.
FIG. 7A is an explanatory figure showing results of observation of the Giemsa staining images of hair follicle mesenchymal cells cultured in the example 2 of Example 2 at a low magnification.
FIG. 7B is an explanatory figure showing results of observation of the Giemsa staining images of the hair follicle mesenchymal cells cultured in the example 2 of Example 2 at a high magnification.
FIG. 8 is an explanatory figure showing micrographs of hair follicle mesenchymal cells (spheroid) cultured in the example C2 of Example 2.
FIG. 9 is an explanatory figure showing results of determination of the numbers of the hair follicle mesenchymal cells cultured in Example 2.
FIG. 10 is an explanatory figure showing results of determination of the ALP gene expression of the hair follicle mesenchymal cells cultured in Example 2.
FIG. 11A is an explanatory figure showing results of observation of dermal papilla cells cultured in the example 3 of Example 3 with a phase-contrast microscope.
FIG. 11B is an explanatory figure showing results of observation of dermal papilla cells (spheroid) cultured in the example C3 of Example 3 with a phase-contrast microscope.
FIG. 12A is an explanatory figure showing results of observation of a cell aggregate containing the dermal papilla cells cultured in the example 3 of Example 3 with a phase-contrast microscope.
FIG. 12B is an explanatory figure showing results of observation of a cell aggregate containing the dermal papilla cells cultured in the example C3 of Example 3 with a phase-contrast microscope.
FIG. 13A is an explanatory figure showing results of transplantation of the cell aggregate containing the dermal papilla cells cultured in the example 3 of Example 3.
FIG. 13B is an explanatory figure showing results of transplantation of the cell aggregate containing the dermal papilla cells cultured in the example C3 of Example 3.
FIG. 14A is an explanatory figure showing results of observation of a hair growing in a site having transplanted thereto the cell aggregate containing the dermal papilla cells cultured in the example 3 of Example 3 with a scanning electron microscope.
FIG. 14B is an explanatory figure showing results of observation of a hair growing in a site having transplanted thereto the cell aggregate containing the dermal papilla cells cultured in the example C3 of Example 3 with a scanning electron microscope.
FIG. 15 is an explanatory figure showing results of determination of the numbers of hairs regenerated in the sites having transplanted thereto the cell aggregates containing the dermal papilla cells in Example 3.
FIG. 16A is an explanatory figure showing a photograph obtained by photographing the transplanted site of a nude mouse on a 21st day after transplantation in the example 4-1 of Example 4.
FIG. 16B is an explanatory figure showing a photograph obtained by photographing the transplanted site of a nude mouse on a 21st day after transplantation in the example 4-2 of Example 4.
FIG. 16C is an explanatory figure showing a photograph obtained by photographing the transplanted site of a nude mouse on a 21st day after transplantation in the example 4C of Example 4.
FIG. 17 is an explanatory figure showing results of counting the numbers of hairs growing in the transplanted sites of the nude mice on the 21st day after the transplantation in Example 4.
FIG. 18 is an explanatory figure showing results of evaluation of relative fluorescence intensities in the transplanted sites of SHO mice in Example 5.

### Description of Embodiments

Now, embodiments of the present invention will be described. It should be noted that the present invention is not limited to these embodiments.

A method according to an embodiment of the present invention (hereinafter referred to as "method of the present invention") encompasses, as one aspect thereof, a method of producing expanded hair follicle mesenchymal cells, the method including a main step of culturing hair follicle mesenchymal cells adhered to a substrate surface from a confluent state without passaging the cells.

The main step of the method of the present invention preferably includes culturing the hair follicle mesenchymal cells to make the cells further from the confluent state until the proliferated hair follicle mesenchymal cells are stacked to form cell layers including two or more stacked layers in at least part of the substrate surface.

In general, for expanding the number of the adhesive cells by two-dimensional culture, the following method is used: first, the cells are seeded onto a cell-adhesive substrate surface at a low density suitable for proliferation of the cells; then, the cells adhered to the substrate surface are cultured and proliferate; and then, the cells are passaged preferably before the cells reach a confluent state, or at the latest, at the time point when the cells reach the confluent state.

The term "passage" refers to the following operation: first, cells are two-dimensionally cultured and proliferate on a substrate surface as described above; then, the cells are detached from the substrate surface and harvested before the cells reach a confluent state or at the time point when the cells reach the confluent state; and then, the harvested cells are seeded again to a new substrate surface. In this regard, for expanding the number of hair follicle mesenchymal cells such as dermal papilla cells in vitro, a method in which the hair follicle mesenchymal cells are two-dimensionally cultured and passaged as described above has heretofore been used.

In contrast, the inventors of the present invention have investigated technical means for achieving both of: the expansion of the number of hair follicle mesenchymal cells (hereinafter referred to as "HFM cells"); and the maintenance or recovery of the hair regeneration abilities of the HFM cells. As a result, the inventors have uniquely and unexpectedly found that, when the HFM cells are cultured to proliferate further from a confluent state without being passaged, the hair regeneration abilities of the HFM cells are effectively recovered while the number of the HFM cells is expanded. Thus, the inventors have completed the present invention.

The HFM cells are mesenchymal cells having hair regeneration abilities. That is, for example, when the HFM cells are transplanted to a living body, the HFM cells promote hair growth in a site to which the HFM cells have been transplanted. Specifically, the HFM cells transplanted to the living body secrete, for example, a factor that promotes the hair growth (e.g., Wnt) to promote the hair growth in the site to which the HFM cells have been transplanted.

The HFM cells may be derived from hair follicles collected from a living body, or may be differentiation-induced in vitro from cells derived from tissues other than the hair follicles. However, the HFM cells are preferably derived from the hair follicles.

Specifically, the HFM cells are preferably mesenchymal cells derived from dermal papillae and/or dermal sheaths in hair follicles collected from a living body, more preferably mesenchymal cells derived from the dermal papillae and/or dermal sheath cups, particularly preferably mesenchymal cells derived from the dermal papillae.

That is, the HFM cells are preferably one or more selected from the group consisting of: dermal papilla cells; and dermal sheath cells, more preferably one or more selected from the group consisting of: the dermal papilla cells; and dermal sheath cup cells, particularly preferably the dermal papilla cells.

Cells to be used in the differentiation induction of the HFM cells are not particularly limited as long as the cells have abilities to differentiate into the HFM cells in vitro. However, the cells are preferably, for example, one or more selected from the group consisting of: pluripotent stem cells (e.g., one or more selected from the group consisting of: induced pluripotent stem (iPS) cells; embryonic stem (ES) cells; multilineage-differentiating-stress-enduring (Muse) cells; and embryonic germ (EG) cells); stem cells other than the pluripotent stem cells (e.g., one or more selected from the group consisting of: stem cells obtained by the reprogramming of differentiated cells; and mesenchymal stem cells (e.g., adipose tissue-derived mesenchymal stem cells)); and mesenchymal cells derived from the skin of an embryo or a neonate (e.g., mesenchymal cells derived from the dermal layer of the skin of the embryo or the neonate).

The HFM cells preferably express one or more hair growth-related genes. The hair growth-related gene to be expressed by the HFM cells is not particularly limited as long as the gene is related to hair growth in a living body. However, the gene may be, for example, one or more selected from the group consisting of: an alkaline phosphatase (ALP) gene; a Versican gene; a LEF1 gene; a WNT5A gene; a NOG gene; a BMP4 gene; a Sox2 gene; an aMSA gene; and a GREM2 gene. The ALP gene is a gene that is strongly expressed particularly by dermal papilla cells, the αMSA gene is a gene to be strongly expressed particularly by dermal sheath cells (including dermal sheath cup cells), and the GREM2 gene is a gene to be strongly expressed particularly by the dermal sheath cup cells. Accordingly, for example, the HFM cells are preferably mesenchymal cells expressing one or more selected from the group consisting of: the ALP gene; the αMSA gene; and the GREM2 gene, more preferably mesenchymal cells expressing one or more selected from the group consisting of: the ALP gene; and the GREM2 gene, particularly preferably mesenchymal cells expressing the ALP gene.

The HFM cells are not particularly limited as long as the cells are derived from an animal having a hair follicle. However, the HFM cells are preferably, for example, the cells of a mammal. The cells of the mammal may be the cells of a human, or may be the cells of a mammal other than a human (e.g., a primate (e.g., a monkey), a rodent (e.g., a mouse, a rat, a hamster, a guinea pig, or a rabbit), a carnivore (e.g., a dog or a cat), or an ungulate (e.g., a pig, a bovine, a horse, a goat, or a sheep)). However, the HFM cells are preferably human cells.

The HFM cells to be used for transplantation to a living body may be derived from an individual to which the HFM cells are transplanted, or may be derived from an individual other than the individual to which the HFM cells are transplanted. However, the HFM cells are preferably derived from the individual to which the cells are transplanted.

For example, human HFM cells to be used for transplantation to a human may be derived from a human patient to whom the human HFM cells are transplanted, or may be derived from a human other than the patient (e.g., human HFM cells differentiation-induced from pluripotent stem cells derived from the human other than the patient (e.g., iPS cells, ES cells, Muse cells, or EG cells stored in a cell bank) in vitro). However, the human HFM cells are preferably derived from the patient to whom the human HFM cells are transplanted.

The number of passages of the HFM cells to be cultured in the method of the present invention is not particularly limited as long as the effects of the present invention are obtained, and HFM cells whose number of passages is zero (HFM cells that have never been passaged) may be used, or HFM cells whose number of passages is 1 or more (HFM cells that have already been passaged once or more) may be used. The number of passages of the HFM cells may be, for example, 15 or less, and is preferably 10 or less, more preferably 8 or less, still more preferably 6 or less, still more preferably 5 or less, still more preferably 4 or less, particularly preferably 3 or less.

Specifically, for example, when the dermal papillae and/or dermal sheaths of the hair follicles collected from a living body (e.g., dermal papillae and/or dermal sheaths subjected to enzymatic treatment after the collection from the living body) are placed on a substrate surface, and HFM cells migrating from the dermal papillae and/or the dermal sheaths onto the substrate surface are obtained, the HFM cells that have migrated onto the substrate surface (HFM cells whose number of passages is zero) may be cultured as they are by the method of the present invention without being detached from the substrate surface. In addition, for example, the following may be performed: the HFM cells whose number of passages is zero are detached from the substrate surface and harvested; and the harvested cells are seeded onto a new substrate surface and cultured as HFM cells whose number of passages is 1, by the method of the present invention. In addition, HFM cells whose number of passages is 2 or more may be cultured by the method of the present invention.

The substrate surface is the surface of a substrate to be used as a culture carrier for the HFM cells, and is a surface having adhesion properties to the HFM cells. A material for forming the substrate surface is not particularly limited as long as the effects of the present invention are obtained. However, the surface is preferably, for example, a surface formed of one or more materials selected from the group consisting of a resin (e.g., polystyrene or polypropylene), glass, a metal, and a ceramic. The substrate surface may be nonporous or porous.

The substrate surface may be subjected to treatment for improving its cell adhesion properties (e.g., coating with a cell-adhesive component), or may not be subjected to the treatment. The cell-adhesive component with which the substrate surface may be coated is not particularly limited as long as the component improves the adhesion properties of the HFM cells to the substrate surface. However, the component is preferably, for example, an extracellular matrix (e.g., collagen or fibronectin), and/or a compound to which a cell-adhesive moiety (e.g., an amino acid sequence including an RGD sequence) has artificially been introduced.

For example, the cell-adhesive surface of a culture vessel (e.g., a culture dish, a culture flask, or a well of a multiwell plate, or a roller bottle) is preferably used as the substrate surface. When the substrate surface of the culture vessel includes a region that is adhesive to the HFM cells and a region that is non-adhesive to the HFM cells, the adhesive region among the surface is used as the substrate surface on which the HFM cells are cultured. In addition, for example, the surface of a culture carrier (e.g., a particulate culture carrier such as microcarrier beads), which is used while being placed on a bottom surface or suspended in a culture solution in a culture vessel, may be used as the substrate surface.

The shape of the substrate surface is not particularly limited as long as the effects of the present invention are obtained. The surface may be a flat surface (e.g., the flat bottom surface of a culture dish, a culture flask, or a well of a multiwell plate), or may be a curved surface (e.g., the inner surface of a roller bottle and/or the surface of a particulate culture carrier such as microcarrier beads).

In the method of the present invention, one substrate surface on which the HFM cells are cultured while being adhered thereto is a continuous cell-adhesive surface to which the HFM cells are adhered. Specifically, the one substrate surface may be, for example, the bottom surface of one culture flask (the bottom surface of one stage in the case of a multistage flask), the inner surface of one roller bottle, the bottom surface of one culture dish, the bottom surface of one well of a multiwell plate, one cell-adhesive surface surrounded by a surface that is non-adhesive to the HFM cells, or the surface of one particle of a particulate carrier (e.g., microcarrier beads).

The area of the one substrate surface is not particularly limited as long as the effects of the present invention are obtained. However, for example, the area may be 5×10⁻⁵ cm² or more, may be 2×10⁻⁴ cm² or more, may be 2×10⁻³ cm² or more, may be 0.01 cm² or more, may be 0.1 cm² or more, may be 0.3 cm² or more, may be 0.5 cm² or more, may be 1 cm² or more, may be 3 cm² or more, may be 5 cm² or more, may be 10 cm² or more, may be 15 cm² or more, or may be 20 cm² or more. In addition, for example, the area of the one substrate surface may be 1,000 cm² or less, may be 800 cm² or less, or may be 600 cm² or less. The area of the one substrate surface may be specified by arbitrarily combining one of the above-mentioned lower limit values and one of the above-mentioned upper limit values.

A culture solution to be used in the culture of the HFM cells is not particularly limited as long as the effects of the present invention are obtained. For example, a culture solution containing a component suitable for the growth of the HFM cells such as a commercially available medium for dermal papilla cell growth is preferably used. Specifically, for example, the culture solution preferably contains one or more selected from the group consisting of: a basic fibroblast growth factor (bFGF); and insulin, and particularly preferably contains the bFGF.

In the main step of the method of the present invention, the HFM cells adhered to the substrate surface in the confluent state are further cultured and made to proliferate without being passaged. That is, the HFM cells in the confluent state are cultured as they are on the substrate surface to further proliferate without being detached from the substrate surface.

The term "confluent state" as used herein refers to a state in which the HFM cells completely cover the entirety of the one substrate surface. Whether or not the HFM cells are in the confluent state can be easily determined by a person having common technical knowledge in the technical field to which the present invention belongs. The confluent state may be specified by, for example, the cell density of the HFM cells on the substrate surface (density obtained by dividing the total number of the HFM cells on the one substrate surface by the area of the one substrate surface). That is, for example, a state in which the HFM cells are confluent may be a state in which the cell density of the HFM cells on the substrate surface falls within the range of from 3×10⁴ cells/cm² or more to 6×10⁴ cells/cm² or less, may be a state in which the density falls within the range of from 3×10⁴ cells/cm² or more to 5.5×10⁴ cells/cm² or less, may be a state in which the density falls within the range of from 3×10⁴ cells/cm² or more to 5×10⁴ cells/cm² or less, may be a state in which the density falls within the range of from 3×10⁴ cells/cm² or more to 4.5×10⁴ cells/cm² or less, or may be a state in which the density falls within the range of from 3×10⁴ cells/cm² or more to 4×10⁴ cells/cm² or less.

The number of the HFM cells on the substrate surface to be used in the calculation of the cell density of the HFM cells on the substrate surface is preferably measured with a measuring kit including: a fluorescent enzyme; and a precursor compound to be converted into a substrate for the fluorescent enzyme by living cells. Specifically, for example, a measuring kit including a luciferase serving as a fluorescent enzyme and a cell membrane-permeable precursor compound to be converted in the cytoplasm of the living cells into a substrate for the luciferase is preferably used. In this case, first, the luciferase and the precursor compound are added to a culture solution containing the HFM cells on the substrate surface. As a result, the precursor compound permeates from the culture solution into the cytoplasm of living HFM cells, and is converted into the substrate for the luciferase by reducing abilities exhibited by the HFM cells in the cytoplasm. Then the produced cell membrane-permeable substrate leaks from the cytoplasm of the HFM cells into the culture solution, and is converted into a light-emitting substance by the luciferase in the culture solution. In view of the foregoing, the intensity (e.g., absorbance at a specific wavelength) of fluorescence derived from the light-emitting substance is measured, and the number of the HFM cells on the substrate surface is calculated based on the measured fluorescence intensity and a calibration curve that has been produced in advance (graph showing a relationship between the fluorescence intensity and the number of cells). The calibration curve is produced by using, for example, the HFM cells at the time of seeding onto the substrate surface. Then, the cell density of the HFM cells on the substrate surface is calculated by dividing the calculated number of the HFM cells by the area of the substrate surface.

The main step preferably includes culturing the HFM cells and make the cells further proliferate from the confluent state, until the proliferated HFM cells are stacked to form cell layers including two or more stacked layers in at least part of the substrate surface.

Specifically, the cell layer, which includes the HFM cells serving as a first layer adhered to the substrate surface and the HFM cells serving as a second layer stacked on the HFM cells serving as the first layer, are formed by making the HFM cells proliferate, without being passaged, further from a confluent state in which the monolayer HFM cells completely cover the substrate surface.

In the main step, the HFM cells may be cultured until the proliferated HFM cells form, in at least part of the substrate surface, the cell layer including three or more, preferably four or more, more preferably five or more, particularly preferably six or more layers of the HFM cells.

In the main step, the HFM cells may be cultured until the proliferated HFM cells form the cell layers including two or more stacked layers in 50% or more of the substrate surface, preferably 60% or more thereof, more preferably 70% or more thereof, still more preferably 80% or more thereof, still more preferably 90% or more thereof, particularly preferably the entire range (100%) of the substrate surface.

In addition, in the main step, the HFM cells may be cultured until the proliferated HFM cells form the cell layers including three or more stacked layers in 20% or more of the substrate surface, preferably 30% or more thereof, more preferably 40% or more thereof, still more preferably 50% or more thereof, still more preferably 60% or more thereof, still more preferably 70% or more thereof, still more preferably 80% or more thereof, particularly preferably 90% or more thereof.

In addition, in the main step, the HFM cells may be cultured until the proliferated HFM cells form the cell layers including four or more stacked layers in 10% or more of the substrate surface, preferably 20% or more thereof, more preferably 30% or more thereof, still more preferably 40% or more thereof, still more preferably 50% or more thereof, particularly preferably 60% or more thereof.

In addition, in the main step, the HFM cells may be cultured until the proliferated HFM cells form the cell layers including five or more stacked layers in 10% or more of the substrate surface, preferably 20% or more thereof, more preferably 30% or more thereof, still more preferably 40% or more thereof, still more preferably 50% or more thereof.

In addition, in the main step, the HFM cells may be cultured until the proliferated HFM cells form the cell layers including six or more stacked layers in 10% or more of the substrate surface, preferably 20% or more thereof, more preferably 30% or more thereof, still more preferably 40% or more thereof, still more preferably 50% or more thereof.

The proliferation of the HFM cells is not necessarily uniform over the entirety of the substrate surface. Accordingly, the number of stacked layers in the cell layers to be formed on the substrate surface may also vary between one part of the substrate surface and another part thereof. Accordingly, in the main step, the HFM cells may be cultured until the proliferated HFM cells form a first cell layer including a first number of stacked HFM cell layers (e.g., one, two, or three HFM cell layers) in one part of the substrate surface, and form a second cell layer including stacked HFM cell layers whose number is larger than the first number by one or more, two or more, or three or more (e.g., four or more, five or more, or six or more HFM cell layers) in another part of the substrate surface.

The cell layers formed by stacking the proliferated HFM cells preferably contain an extracellular matrix (e.g., type I collagen and/or fibronectin) secreted by the HFM cells. That is, the cell layers preferably include two or more HFM cell layers, and the extracellular matrix being secreted by the HFM cells and accumulated between the HFM cells.

The main step preferably includes culturing the HFM cells without passaging the cells until a time period of 120 hours or more elapses from the confluent state. Specifically, for example, the HFM cells are continuously cultured on the substrate surface without being passaged for an additional 120 hours or more from the time point when the HFM cells reach the confluent state on the substrate surface (e.g., the time point when the density of the cells reaches the cell density at which the above-mentioned confluent state is formed).

In this case, the main step may include culturing the HFM cells without passaging the cells until a time period of 200 hours or more, preferably 300 hours or more, more preferably 400 hours or more, still more preferably 500 hours or more, still more preferably 550 hours or more, particularly preferably 600 hours or more elapses from the confluent state.

In addition, for example, the time period for which the HFM cells are cultured from the confluent state without being passaged in the main step may be 2,400 hours or less, may be 2,000 hours or less, may be 1,800 hours or less, may be 1,600 hours or less, may be 1,400 hours or less, may be 1,200 hours or less, may be 950 hours or less, or may be 700 hours or less. The time period for which the HFM cells are cultured from the confluent state without being passaged in the main step may be specified by arbitrarily combining one of the above-mentioned lower limit values and one of the above-mentioned upper limit values.

The main step preferably includes culturing the HFM cells without passaging the HFM cells until the cell density of the HFM cells on the substrate surface reaches 6.5×10⁴ cells/cm² or more. In this case, the main step may include culturing the HFM cells until the cell density of the HFM cells on the substrate surface reaches 7×10⁴ cells/cm² or more, preferably 7.5×10⁴ cells/cm² or more, more preferably 8×10⁴ cells/cm² or more, still more preferably 8.5×10⁴ cells/cm² or more, still more preferably 9×10⁴ cells/cm² or more, still more preferably 9.5×10⁴ cells/cm² or more, particularly preferably 10×10⁴ cells/cm² or more.

In addition, for example, the cell density reached in the main step by culturing the HFM cells from the confluent state without passaging the cells may be 25×10⁴ cells/cm² or less, may be 20×10⁴ cells/cm² or less, may be 18×10⁴ cells/cm² or less, may be 16×10⁴ cells/cm² or less, may be 15×10⁴ cells/cm² or less, may be 14×10⁴ cells/cm² or less, may be 13×10⁴ cells/cm² or less, or may be 12×10⁴ cells/cm² or less. The cell density reached in the main step by culturing the HFM cells from the confluent state without passaging the cells may be specified by arbitrarily combining one of the above-mentioned lower limit values and one of the above-mentioned upper limit values.

The main step preferably includes culturing the HFM cells for 50 hours or more in a state where the cell density of the HFM cells on the substrate surface is 7×10⁴ cells/cm² or more. In this case, the main step may include culturing the HFM cells for 100 hours or more, preferably 200 hours or more, more preferably 300 hours or more, still more preferably 350 hours or more, still more preferably 400 hours or more, particularly preferably 450 hours or more, in a state where the cell density of the HFM cells on the substrate surface is 7×10⁴ cells/cm² or more.

In addition, for example, the time period for which the culture is performed in a state where the cell density is 7×10⁴ cells/cm² or more in the main step may be 2,000 hours or less, may be 1,500 hours or less, may be 1,000 hours or less, may be 800 hours or less, may be 600 hours or less, or may be 500 hours or less. The time period for which the culture is performed in a state where the cell density is 7×10⁴ cells/cm² or more in the main step may be specified by arbitrarily combining one of the above-mentioned lower limit values and one of the above-mentioned upper limit values.

The main step may include culturing the HFM cells for 50 hours or more, preferably 150 hours or more, more preferably 250 hours or more, still more preferably 300 hours or more, particularly preferably 350 hours or more in a state where the cell density of the HFM cells on the substrate surface is 9×10⁴ cells/cm² or more.

In addition, for example, the time period for which the culture is performed in a state where the cell density of the HFM cells on the substrate surface is 9×10⁴ cells/cm² or more in the main step may be 2,000 hours or less, may be 1,500 hours or less, may be 1,000 hours or less, may be 800 hours or less, may be 600 hours or less, may be 500 hours or less, or may be 400 hours or less. The time period for which the culture is performed in a state where the cell density of the HFM cells on the substrate surface is 9×10⁴ cells/cm² or more in the main step may be specified by arbitrarily combining one of the above-mentioned lower limit values and one of the above-mentioned upper limit values.

The main step may include culturing the HFM cells for 50 hours or more, preferably 100 hours or more, more preferably 150 hours or more, still more preferably 200 hours or more, particularly preferably 250 hours or more in a state where the cell density of the HFM cells on the substrate surface is 10×10⁴ cells/cm² or more.

In addition, for example, the time period for which the culture is performed in a state where the cell density of the HFM cells on the substrate surface is 10×10⁴ cells/cm² or more in the main step may be 2,000 hours or less, may be 1,500 hours or less, may be 1,000 hours or less, may be 500 hours or less, may be 400 hours or less, or may be 300 hours or less. The time period for which the culture is performed in a state where the cell density of the HFM cells on the substrate surface is 10×10⁴ cells/cm² or more in the main step may be specified by arbitrarily combining one of the above-mentioned lower limit values and one of the above-mentioned upper limit values.

The main step preferably includes culturing the HFM cells until the cell density of the HFM cells on the substrate surface reaches 1.5 or more times as high as that in the confluent state. In this case, the main step may include culturing the HFM cells until the cell density of the HFM cells on the substrate surface reaches twice or more, preferably 2.5 or more times, more preferably 3 or more times, particularly preferably 3.5 or more times as high as that in the confluent state.

The cell density of the HFM cells reached in the main step may be 10 or less times, may be 8 or less times, may be 6 or less times, may be 5 or less times, or may be 4 or less times as high as the cell density in the confluent state. The cell density of the HFM cells reached in the main step may be specified by arbitrarily combining one of the above-mentioned lower limit values and one of the above-mentioned upper limit values.

The main step preferably includes culturing the HFM cells until the expression amount of each of the above-mentioned one or more hair growth-related genes (e.g., the ALP gene) of the HFM cells increases to 1.5 or more times as large as that in the confluent state. In this case, the main step may include culturing the HFM cells until the expression amount of each of the one or more hair growth-related genes of the HFM cells increases to 2.0 or more times, preferably 2.5 or more times, more preferably 3.0 or more times, still more preferably 3.5 or more times, still more preferably 4.0 or more times, still more preferably 4.5 or more times, still more preferably 5.0 or more times, particularly preferably 5.5 or more times as large as that in the confluent state.

In addition, the expression amount of each of the hair growth-related genes reached in the main step by culturing the HFM cells may be 100 or less times, may be 50 or less times, or may be 40 or less times as large as that in the confluent state. The expression amount of each of the hair growth-related genes reached in the main step by culturing the HFM cells may be specified by arbitrarily combining one of the above-mentioned lower limit values and one of the above-mentioned upper limit values.

In the method of the present invention, a method of forming the confluent state of the HFM cells on the substrate surface is not particularly limited as long as the effects of the present invention are obtained. However, the confluent state may be formed by, for example, seeding the HFM cells onto the substrate surface at the cell density at which the confluent state is formed in the main step. Alternatively, the confluent state may be formed by culturing the HFM cells that have not reached the confluent state to make the cells proliferate on the substrate surface before the performance of the main step.

That is, the method of the present invention further includes a pre-step to be performed prior to the main step, and the pre-step may include culturing the HFM cells that have not yet reached the confluent state on the substrate surface to make the HFM cells proliferate until the HFM cells reach the confluent state.

In this case, also in the pre-step, the HFM cells adhered to the substrate surface are cultured without being passaged. That is, in the method of the present invention, throughout the pre-step and the main step, the HFM cells are cultured without being passaged. Specifically, first, in the pre-step, the HFM cells that have not yet reached the confluent state on the substrate surface proliferate until the HFM cells reach the confluent state on the substrate surface by culturing the HFM cells on the substrate surface without passaging the cells. Then, in the main step subsequent to the pre-step, the HFM cells that have already reached the confluent state on the substrate surface in the pre-step are cultured on the substrate surface to further proliferate without being passaged.

For example, the culture of the HFM cells that have not yet reached the confluent state in the pre-step may be the culture of the HFM cells on the substrate surface, at least part of which has not yet been covered with any cells, and/or may be the culture of the HFM cells at a cell density smaller than the cell density at which the confluent state is formed on the substrate surface.

The pre-step may include culturing the HFM cells at a cell density of 2.5×10⁴ cells/cm² or less to make the HFM cells proliferate until the cells reach the confluent state. In this case, the pre-step may include culturing the HFM cells at a cell density of preferably 2×10⁴ cells/cm² or less, more preferably 1.5×10⁴ cells/cm² or less, still more preferably 1×10⁴ cells/cm² or less, still more preferably 0.8×10⁴ cells/cm² or less, particularly preferably 0.7×10⁴ cells/cm² or less to make the HFM cells proliferate until the cells reach the confluent state.

In addition, the pre-step may include culturing the HFM cells at a cell density of 0.005×10⁴ cells/cm² or more, 0.01×10⁴ cells/cm² or more, 0.05×10⁴ cells/cm² or more, 0.1×10⁴ cells/cm² or more, 0.2×10⁴ cells/cm² or more, 0.3×10⁴ cells/cm² or more, 0.4×10⁴ cells/cm² or more, or 0.5×10⁴ cells/cm² or more, to make the HFM cells proliferate until the cells reach the confluent state. The cell density at which the HFM cells are cultured in the pre-step may be specified by arbitrarily combining one of the above-mentioned lower limit values and one of the above-mentioned upper limit values.

The pre-step may further include seeding the HFM cells onto the substrate surface. That is, the pre-step may include: firstly seeding the HFM cells onto the substrate surface at the cell density at which the HFM cells do not reach the confluent state; and then culturing the HFM cells adhered to the substrate surface to make the HFM cells proliferate until the cells reach the confluent state.

In this case, the pre-step may include seeding the HFM cells onto the substrate surface at a cell density of 2.5×10⁴ cells/cm² or less, preferably 2×10⁴ cells/cm² or less, more preferably 1.5×10⁴ cells/cm² or less, still more preferably 1×10⁴ cells/cm² or less, still more preferably 0.8×10⁴ cells/cm² or less, particularly preferably 0.7×10⁴ cells/cm² or less.

In addition, the pre-step may include seeding the HFM cells onto the substrate surface at a cell density of 0.005×10⁴ cells/cm² or more, 0.01×10⁴ cells/cm² or more, 0.05×10⁴ cells/cm² or more, 0.1×10⁴ cells/cm² or more, 0.2×10⁴ cells/cm² or more, 0.3×10⁴ cells/cm² or more, 0.4×10⁴ cells/cm² or more, or 0.5×10⁴ cells/cm² or more. The seeding density of the HFM cells in the pre-step may be specified by arbitrarily combining one of the above-mentioned lower limit values and one of the above-mentioned upper limit values.

In the method of the present invention, the HFM cells dispersed in a culture solution may be seeded onto the substrate surface. Herein, each HFM cell dispersed in the culture solution is substantially free from being adhered to any other cell, or adheres to the other cell, but is easily separated from the other cell by fluidizing the culture solution through an operation such as pipetting.

In addition, in the method of the present invention, the HFM cells for forming a cell aggregate may be seeded onto the substrate surface. That is, for example, the following may be performed: the cell aggregate (e.g., spheroid) of the HFM cells is formed in advance; and then, a culture solution containing the cell aggregate is added onto the substrate surface to seed the HFM cells which form the cell aggregate onto the substrate surface. In this case, some HFM cells out of the HFM cells which form the cell aggregate are adhered to the substrate surface, and migrate onto the substrate surface. Then, when the HFM cells that have migrated from the cell aggregate to the substrate surface are cultured on the substrate surface, the HFM cells can proliferate on the substrate surface. Specifically, for example, the following may be performed in the pre-step: the cell aggregate formed of the HFM cells are seeded onto the substrate surface; and the HFM cells that have migrated from the cell aggregate to the substrate surface, at least part of which has not yet been covered with any cell, are cultured and proliferate on the substrate surface. The formation of the cell aggregate containing the HFM cells will be described later.

While the culture time of the HFM cells in the pre-step (i.e., a time period from the time point when the culture of the HFM cells is started in the pre-step (e.g., from the time point when the HFM cells are seeded onto the substrate surface in the pre-step) to the time point when the cells reach the confluent state) is not particularly limited as long as the effects of the present invention are obtained, for example, the culture time may be 20 hours or more, may be 40 hours or more, may be 60 hours or more, may be 80 hours or more, or may be 100 hours or more.

In addition, for example, the culture time of the HFM cells in the pre-step may be 500 hours or less, may be 450 hours or less, may be 400 hours or less, may be 350 hours or less, may be 300 hours or less, may be 250 hours or less, may be 200 hours or less, or may be 150 hours or less. The culture time of the HFM cells in the pre-step may be specified by arbitrarily combining one of the above-mentioned lower limit values and one of the above-mentioned upper limit values.

In the case where the method of the present invention includes the pre-step and the main step, the main step may include culturing the HFM cells until a time period of 200 hours or more elapses from the start of the culture of the HFM cells in the pre-step (e.g., the time point when the HFM cells are seeded onto the substrate surface in the pre-step). In this case, the main step may include culturing the HFM cells until a time period of preferably 250 hours or more, more preferably 300 hours or more, still more preferably 350 hours or more, still more preferably 400 hours or more, still more preferably 450 hours or more, still more preferably 500 hours or more, still more preferably 550 hours or more, still more preferably 600 hours or more, still more preferably 650 hours or more, particularly preferably 700 hours or more, elapses from the start of the culture of the HFM cells in the pre-step.

In addition, the main step may include culturing the HFM cells until a time period of 2,500 hours or less, 2,000 hours or less, 1,800 hours or less, 1,600 hours or less, 1,400 hours or less, 1,200 hours or less, 1,000 hours or less, 900 hours or less, 850 hours or less, 800 hours or less, or 750 hours or less, elapses from the start of the culture of the HFM cells in the pre-step. The time period for which the HFM cells are cultured in the main step may be specified by arbitrarily combining one of the above-mentioned lower limit values and one of the above-mentioned upper limit values.

In the case where the method of the present invention includes the pre-step and the main step, the main step may include culturing the HFM cells until the expression amount of each of the above-mentioned one or more hair growth-related genes (e.g., the ALP gene) of the HFM cells increases to 1.1 or more times as large as that at the time point when 24 hours elapses from the start of the culture of the HFM cells in the pre-step (e.g., the time point when the HFM cells are seeded onto the substrate surface in the pre-step). In this case, the main step may include culturing the HFM cells until the expression amount of each of the one or more hair growth-related genes of the HFM cells increases to 1.2 or more times, preferably 1.5 or more times, more preferably twice or more, still more preferably 2.5 or more times, still more preferably 3 or more times, still more preferably 3.5 or more times, particularly preferably 3.8 or more times as large as that at the time point when 24 hours elapses from the start of the culture of the HFM cells in the pre-step.

In addition, the expression amount of each of the hair growth-related genes of the HFM cells reached in the main step may be 50 or less times, may be 30 or less times, may be 25 or less times, may be 20 or less times, may be 15 or less times, or may be 10 or less times as large as that at the time point when 24 hours elapses from the start of the culture of the HFM cells in the pre-step. The expression amount of each of the hair growth-related genes of the HFM cells reached in the main step may be specified by arbitrarily combining one of the above-mentioned lower limit values and one of the above-mentioned upper limit values.

In the method of the present invention, while a ratio of the number of the HFM cells, which are included in cells to be seeded onto the substrate surface or cells to be cultured on the substrate surface, to the total number of the cells is not particularly limited as long as the effects of the present invention are obtained, the ratio may be, for example, 80% or more, and is preferably 90% or more, particularly preferably 95% or more.

In the method of the present invention, for example, a ratio of the number of dermal papilla cells, which are included in the HFM cells to be seeded onto the substrate surface or the HFM cells to be cultured on the substrate surface, to the total number of the HFM cells, may be 5% or more, may be 10% or more, may be 20% or more, may be 30% or more, may be 40% or more, may be 50% or more, may be 60% or more, may be 70% or more, may be 80% or more, may be 90% or more, or may be 95% or more.

The method of the present invention may further include a harvesting step of detaching the HFM cells, which have been obtained through the culture on the substrate surface from the confluent state without the passage in the main step, from the substrate surface, followed by harvesting the detached HFM cells.

That is, in this case, first, in the main step, the HFM cells whose number has been expanded (hereinafter referred to as "expanded HFM cells") are obtained by culturing the HFM cells from the confluent state on the substrate surface without passaging the cells. Next, in the harvesting step subsequent to the main step, the expanded HFM cells obtained in the main step are detached from the substrate surface and harvested.

Specifically, in the harvesting step, for example, the expanded HFM cells are harvested as dispersed cells. That is, for example, the expanded HFM cells adhered onto the substrate surface are subjected to enzymatic treatment so that the expanded HFM cells are detached from the substrate surface and dispersed in a culture solution, followed by harvesting the expanded HFM cells dispersed in the culture solution. For example, a protease such as trypsin is preferably used in the enzymatic treatment.

In addition, in the harvesting step, for example, the expanded HFM cells are harvested as dispersed cell layer pieces. That is, one or more cell layer pieces dispersed in the culture solution are harvested by, for example, crushing or detaching a cell layer of the expanded HFM cells formed on the substrate surface.

More specifically, for example, the following may be performed: the cell layer of the expanded HFM cells covering the substrate surface is crushed with a crushing tool such as a cell scraper, followed by harvesting a plurality of cell layer pieces formed by crushing the cell layers and dispersed in the culture solution.

In addition, the plurality of cell layer pieces dispersed in the culture solution may be harvested by, for example, forming a plurality of cell layers of the expanded HFM cells separated from each other on the substrate surface, and then detaching each of the plurality of cell layers from the substrate surface. In this case, a method of forming the plurality of cell layers separated from each other on the substrate surface is not particularly limited as long as the effects of the present invention are obtained. However, there may be used, for example, a method including: forming, on one substrate surface, a plurality of regions that are adhesive to the HFM cells, the regions each being surrounded by a surface that is non-adhesive to the HFM cells; and culturing the HFM cells in the respective adhesive regions on the substrate surface to form cell layers.

A method of detaching one or more cell layers from the substrate surface is not particularly limited as long as the effects of the present invention are obtained. However, for example, the following may be performed: only the outer peripheral portions of the cell layers are peeled with a peeling tool such as a cell scraper in the culture solution so that the peeling of the outer peripheral portions of the cell layers and the subsequent aggregation of the cell layers are promoted; and finally, the aggregated body of the cell layers is detached from the substrate surface.

In addition, there may be used, for example, a method including: culturing the HFM cells on a cell-adhesive substrate surface coated with a temperature-responsive polymer to form a cell layer; and then changing a temperature to solubilize the temperature-responsive polymer, to thereby detach the cell layer from the substrate surface. In addition, such an electrochemical method as described in, for example, JP 2008-295382 A may be used.

The harvested cell layer pieces include expanded HFM cell layers having two or more laminated layers, the expanded HFM cell layers being derived from the cell layers formed on the substrate surface. In addition, the harvested cell layer pieces preferably further include an extracellular matrix, which is secreted from the HFM cells and accumulated in the cell layers.

As described above, according to the method of the present invention including at least the main step, the number of the HFM cells is expanded, and moreover, the hair regeneration abilities of the HFM cells are effectively recovered. That is, according to the method of the present invention, expanded HFM cells having high hair regeneration abilities are produced.

The expanded HFM cells to be finally obtained in the main step of the method of the present invention (i.e., expanded HFM cells to be obtained by long-term and high-density culture in the main step) have hair regeneration abilities higher than those of the HFM cells in the confluent state before the culture for obtaining the expanded HFM cells.

That is, for example, when the expanded HFM cells produced by the method of the present invention are transplanted to a living body, the cells have abilities to regenerate, in the transplanted site of the living body, a larger number of hairs than the HFM cells harvested from the confluent state before the culture for obtaining the expanded HFM cells.

Specifically, the main step of the method of the present invention may include obtaining expanded HFM cells having, for example, the following abilities: when the expanded HFM cells are transplanted to a living body, the expanded HFM cells regenerate, in the transplanted site of the living body, hairs numbering 5 or more times, preferably 10 or more times, more preferably 15 or more times, still more preferably 20 or more times, still more preferably 25 or more times, particularly preferably 30 or more times, as many as hairs generated by HFM cells harvested from a confluent state.

In view of the foregoing, the method of the present invention encompasses, as another aspect, a hair regeneration method, the method including transplanting, to a living body, expanded HFM cells produced as described above. The living body to which the expanded HFM cells are transplanted is not particularly limited as long as the living body is an animal having a hair follicle. However, for example, the living body is preferably a mammal, particularly preferably a human. The transplantation of the expanded HFM cells to the living body is preferably transplantation to the skin of the living body, particularly preferably transplantation to the scalp of the living body.

The transplantation of the expanded HFM cells to the living body, which may be intended for medical applications or research applications, is preferably intended for the treatment or prevention of a disease involving hair loss. That is, the transplantation of the expanded HFM cells to the living body is preferably transplantation to a human patient suffering from the disease involving hair loss, or a human patient who is at a risk of suffering from the disease involving hair loss. Accordingly, the hair regeneration method including transplanting the expanded HFM cells to the living body is preferably a method of treating or preventing the disease involving hair loss.

The disease involving hair loss is not particularly limited, but may be, for example, one or more selected from the group consisting of: androgenetic alopecia (AGA); female androgenetic alopecia (FAGA); postpartum alopecia; diffuse alopecia; seborrheic alopecia; alopecia pityrodes; traction alopecia; metabolic alopecia; pressure alopecia; alopecia areata; alopecia neurotica; hair-pulling disorder; universalis alopecia; and symptomatic alopecia.

At the time of the transplantation of the expanded HFM cells to the living body, a composition for transplantation containing the expanded HFM cells is preferably transplanted to the living body. In this respect, the method of the present invention encompasses, as still another aspect, a method of producing a composition for transplantation, the method including preparing a composition for transplantation containing expanded HFM cells produced as described above.

The composition for transplantation containing the expanded HFM cells is not particularly limited as long as an effect exhibited by its transplantation is obtained. However, the composition preferably contains, for example, the expanded HFM cells and a biocompatible carrier that maintains the survival of the expanded HFM cells. Specifically, the carrier in the composition for transplantation preferably contains, for example, an isotonic solution such as physiological saline. The composition for transplantation may further contain any other component. That is, the composition for transplantation may further contain, for example, an extracellular matrix component such as collagen.

The forms of the expanded HFM cells in the composition for transplantation are not particularly limited as long as an effect exhibited by its transplantation is obtained. However, the composition for transplantation preferably contains, for example, expanded HFM cells that are dispersed and/or a plurality of expanded HFM cells adhered to each other.

The expanded HFM cells that are dispersed are obtained by, for example, subjecting the expanded HFM cells adhered onto the substrate surface to enzymatic treatment. The plurality of expanded HFM cells that are adhered to each other are obtained as, for example, the cell layer pieces and/or cell aggregate of the expanded HFM cells. As described above, the cell layer pieces are obtained by, for example, harvesting the cell layers of the expanded HFM cells formed on the substrate surface in the main step. The cell aggregate is formed by aggregating the expanded HFM cells that are dispersed and/or the cell layers of the expanded HFM cells formed on the substrate surface. The cell aggregate containing the HFM cells may be a spherical cell aggregate (spheroid).

In this respect, the method of the present invention encompasses, as still another aspect, a method of producing a cell aggregate, the method including forming a cell aggregate containing expanded HFM cells produced as described above. In the method of producing a cell aggregate, the expanded HFM cells are aggregated to form the cell aggregate containing the expanded HFM cells.

A method of aggregating the expanded HFM cells is not particularly limited as long as the effects of the present invention are obtained. However, for example, a method including subjecting the expanded HFM cells to suspension culture, a method including subjecting the expanded HFM cells to centrifugation treatment, or a method including peeling the cell layers of the expanded HFM cells from the substrate surface to aggregate the cells is preferably used.

That is, for example, the expanded HFM cells being dispersed are cultured in a suspended state (non-adherent state) so that the expanded HFM cells may be aggregated to form the cell aggregate containing the expanded HFM cells. The suspension culture is performed in a culture solution having fluidity as a whole. In addition, a culture vessel having a surface that is non-adhesive to the cells (e.g., a culture dish, a culture flask, or a well of a multiwell plate) is preferably used with the suspension culture.

In this case, in the suspension culture, seeded cells are cultured in a non-adherent state while being substantially free from being adhered to the surface of the culture vessel. Specifically, for example, the cells that have precipitated on the non-adhesive surface of the culture vessel are not adhered to the surface in the culture solution, or adhere to the surface to such a weak extent as to be easily detached from the surface by fluidizing the culture solution through an operation such as pipetting. The shapes of the cells cultured on the non-adhesive bottom surface of the vessel are maintained substantially spherical.

In addition, the cell aggregate is formed in the state of being suspended in the culture solution (i.e., a non-adhesive state) while being substantially free from being adhered to the non-adhesive surface. Specifically, for example, the cell aggregate formed in the culture vessel having the non-adhesive surface is not adhered to the surface in the culture solution, or adheres to the surface to such a weak extent as to be easily detached from the surface by fluidizing the culture solution through an operation such as pipetting.

In addition, for example, the following may be performed: the expanded HFM cells that are dispersed in the culture solution in the vessel are subjected to centrifugation treatment so that the expanded HFM cells may be precipitated and aggregated in the vessel to form the cell aggregate containing the expanded HFM cells.

In addition, for example, as described above, the cell aggregate may be formed by peeling only the outer peripheral portions of the cell layers formed on the substrate surface with a peeling tool such as a cell scraper in the culture solution to promote the peeling of the outer peripheral portions of the cell layers and subsequent aggregation of the cell layers.

When the cell aggregate containing the HFM cells is spherical, the diameter of the cell aggregate is not particularly limited as long as the effects of the present invention are obtained. However, the diameter may be, for example, 20 um or more, and is preferably 40 um or more, more preferably 60 um or more, still more preferably 80 um or more, particularly preferably 100 um or more. In addition, the diameter of the cell aggregate containing the HFM cells may be, for example, 900 um or less, and is preferably 800 um or less, more preferably 700 um or less, still more preferably 600 um or less, particularly preferably 500 um or less. The diameter of the cell aggregate containing the HFM cells may be specified by arbitrarily combining one of the above-mentioned lower limit values and one of the above-mentioned upper limit values.

The number of cells to be used in the formation of one cell aggregate and/or the number of the cells in the one cell aggregate is not particularly limited as long as the effects of the present invention are obtained. However, each of the numbers may be, for example, 0.1×10³ or more, and is preferably 0.5×10³ or more, more preferably 0.6×10³ or more, still more preferably 0.7×10³ or more, still more preferably 0.8×10³ or more, still more preferably 0.9×10³ or more, particularly preferably 1.0×10³ or more.

In addition, for example, the number of cells to be used in the formation of one cell aggregate and/or the number of the cells in the one cell aggregate may be 15×10⁴ or less, may be 10×10⁴ or less, may be 8×10⁴ or less, may be 7×10⁴ or less, may be 6×10⁴ or less, may be 5×10⁴ or less, may be 4×10⁴ or less, may be 3×10⁴ or less, may be 2×10⁴ or less, or may be 1×10⁴ or less. The number of the cells to be used in the formation of the one cell aggregate and the number of the cells in the one cell aggregate may each be specified by arbitrarily combining one of the above-mentioned lower limit values and one of the above-mentioned upper limit values.

A ratio of the number of the HFM cells, which are included in the cells to be used in the formation of the cell aggregate, to the total number of the cells is not particularly limited as long as the effects of the present invention are obtained. However, for example, the ratio may be 10% or more, may be 20% or more, may be 30% or more, may be 40% or more, may be 50% or more, may be 60% or more, may be 70% or more, may be 80% or more, may be 90% or more, or may be 95% or more.

In addition, a ratio of the number of the HFM cells, which are included in the cells in the cell aggregate, to the total number of the cells, is not particularly limited as long as the effects of the present invention are obtained. However, for example, the ratio may be 10% or more, may be 20% or more, may be 30% or more, may be 40% or more, may be 50% or more, may be 60% or more, may be 70% or more, may be 80% or more, may be 90% or more, or may be 95% or more.

A ratio of the number of dermal papilla cells, which are included in the HFM cells to be used in the formation of the cell aggregate, to the number of the HFM cells, is not particularly limited as long as the effects of the present invention are obtained. However, for example, the ratio may be 10% or more, may be 20% or more, may be 30% or more, may be 40% or more, may be 50% or more, may be 60% or more, may be 70% or more, may be 80% or more, may be 90% or more, or may be 95% or more.

In addition, a ratio of the number of the dermal papilla cells, which are included in the HFM cells in the cell aggregate, to the number of the HFM cells is not particularly limited as long as the effects of the present invention are obtained. However, for example, the ratio may be 10% or more, may be 20% or more, may be 30% or more, may be 40% or more, may be 50% or more, may be 60% or more, may be 70% or more, may be 80% or more, may be 90% or more, or may be 95% or more.

In the case where the cells to be used in the formation of the cell aggregate further include other cells in addition to the expanded HFM cells, the other cells are not particularly limited as long as the effects of the present invention are obtained. However, the other cells are preferably, for example, epithelial cells.

In this case, for example, a ratio between the number of the expanded HFM cells to be used in the formation of the cell aggregate and the number of the epithelial cells to be used in the formation (expanded HFM cells:epithelial cells) may fall within the range of from 1:10 to 10:1, may fall within the range of from 1:9 to 9:1, may fall within the range of from 1:8 to 8:1, may fall within the range of from 1:7 to 7:1, may fall within the range of from 1:6 to 6:1, may fall within the range of from 1:5 to 5:1, may fall within the range of from 1:4 to 4:1, may fall within the range of from 1:3 to 3:1, or may fall within the range of from 1:2 to 2:1.

In addition, for example, a ratio between the number of the expanded HFM cells in the cell aggregate and the number of the epithelial cells therein (expanded HFM cells:epithelial cells) may fall within the range of from 1:10 to 10:1, may fall within the range of from 1:9 to 9:1, may fall within the range of from 1:8 to 8:1, may fall within the range of from 1:7 to 7:1, may fall within the range of from 1:6 to 6:1, may fall within the range of from 1:5 to 5:1, may fall within the range of from 1:4 to 4:1, may fall within the range of from 1:3 to 3:1, or may fall within the range of from 1:2 to 2:1.

The epithelial cells to be used in the formation of the cell aggregate are not particularly limited as long as the effects of the present invention are obtained. However, the epithelial cells are preferably, for example, one or more selected from the group consisting of: hair follicle epithelial cells; and precursor cells of the hair follicle epithelial cells. The hair follicle epithelial cells are epithelial cells that contribute to hair growth (more specifically, for example, epithelial cells that contribute to the hair growth in coordination with HFM cells).

The hair follicle epithelial cells may be derived from hair follicles collected from a living body, or may be differentiation-induced in vitro. However, the cells are preferably derived from the hair follicles collected from the living body.

Specifically, the hair follicle epithelial cells are preferably one or more selected from the group consisting of: hair follicle epithelial stem cells; hair matrix cells; outer root sheath cells; and inner root sheath cells, particularly preferably one or more selected from the group consisting of: the hair follicle epithelial stem cells; the hair matrix cells; and the outer root sheath cells.

Undifferentiated cells to be used in the differentiation induction of the hair follicle epithelial cells are not particularly limited as long as the cells have abilities to differentiate into the hair follicle epithelial cells in vitro. However, the undifferentiated cells are preferably, for example, one or more selected from the group consisting of: pluripotent stem cells (e.g., iPS cells, ES cells, Muse cells, or EG cells); and stem cells other than the pluripotent stem cells (e.g., stem cells obtained by the reprogramming of differentiated cells).

The precursor cells of the hair follicle epithelial cells are not particularly limited as long as the cells have abilities to differentiate into the hair follicle epithelial cells in vitro. However, the precursor cells are preferably, for example, one or more selected from the group consisting of: the skin epithelial cells of an embryo or a neonate (e.g., epithelial cells derived from the epidermal layer of the skin of the embryo or the neonate); and stem cells other than pluripotent stem cells having abilities to differentiate into the hair follicle epithelial cells in vitro (e.g., the basal cells of the skin).

The hair follicle epithelial cells are not particularly limited as long as the cells are derived from an animal having a hair follicle. However, the hair follicle epithelial cells are preferably, for example, the cells of a mammal. The cells of the mammal may be the cells of a human, or may be the cells of a mammal other than a human (e.g., a primate (e.g., a monkey), a rodent (e.g., a mouse, a rat, a hamster, a guinea pig, or a rabbit), a carnivoran (e.g., a dog or a cat), or an ungulate (e.g., a pig, a bovine, a horse, a goat, or a sheep)). However, the hair follicle epithelial cells are preferably human cells.

The hair follicle epithelial cells to be used for transplantation to a living body may be derived from an individual to which the hair follicle epithelial cells are transplanted, or may be derived from an individual other than the individual to which the hair follicle epithelial cells are transplanted. However, the hair follicle epithelial cells are preferably derived from the individual to which the hair follicle epithelial cells are transplanted.

For example, human hair follicle epithelial cells to be used for transplantation to a human may be derived from a human patient to whom the human hair follicle epithelial cells are transplanted, or may be cells derived from a human other than the patient (e.g., human cells differentiation-induced from pluripotent stem cells derived from a human other than the patient (e.g., iPS cells, ES cells, Muse cells, or EG cells stored in a cell bank) in vitro). However, the human hair follicle epithelial cells are preferably derived from the human patient to whom the human hair follicle epithelial cells are transplanted.

The cell aggregate containing the expanded HFM cells and the epithelial cells may include: a HFM cell-aggregated portion formed by the aggregation of the expanded HFM cells; and an epithelial cell-aggregated portion formed by the aggregation of the epithelial cells.

That is, the cell aggregate including the HFM cell-aggregated portion and the epithelial cell-aggregated portion may be formed, for example, as follows: dispersed cells including the expanded HFM cells and the epithelial cells are subjected to suspension culture in a culture solution so that the expanded HFM cells aggregate to form the HFM cell-aggregated portion, and the epithelial cells aggregate to form the epithelial cell-aggregated portion; and part of the HFM cell-aggregated portion and part of the epithelial cell-aggregated portion are adhered to form the aggregate.

In addition, the cell aggregate including the HFM cell-aggregated portion and the epithelial cell-aggregated portion may be formed by, for example, separately performing the formation of the HFM cell-aggregated portion through the centrifugation treatment of the expanded HFM cells and the formation of the epithelial cell-aggregated portion through the centrifugation treatment of the epithelial cells, and then bringing the HFM cell-aggregated portion and the epithelial cell-aggregated portion into contact with each other to adhere the portions.

In addition, the cell aggregate including the HFM cell-aggregated portion and the epithelial cell-aggregated portion that are laminated may be formed by, for example, subjecting one of the expanded HFM cells and the epithelial cells to centrifugation treatment in a vessel to form one of the HFM cell-aggregated portion and the epithelial cell-aggregated portion, then adding the other of the proliferated HFM cells and the epithelial cells onto the portion, and subjecting the cells to centrifugation treatment to form the other of the HFM cell-aggregated portion and the epithelial cell-aggregated portion.

The cell aggregate containing the expanded HFM cells has a hair regeneration ability. The hair regeneration ability of the cell aggregate is such an ability that when the cell aggregate is transplanted to a living body, hair growth is caused in a site to which the cell aggregate is transplanted.

That is, for example, when the cell aggregate containing the expanded HFM cells is transplanted to a living body, the aggregate has an ability to regenerate, in the transplanted site of the living body, a larger number of hairs than a cell aggregate containing, instead of the expanded HFM cells, HFM cells harvested from a confluent state before culture for obtaining the expanded HFM cells.

Specifically, the method of producing a cell aggregate containing expanded HFM cells may include obtaining a cell aggregate having, for example, the following ability: when the aggregate is transplanted to a living body, the aggregate regenerates, in the transplanted site of the living body, hairs numbering 5 or more times, preferably 10 or more times, more preferably 15 or more times, still more preferably 20 or more times, still further more preferably 25 or more times, particularly preferably 30 or more times as many as hairs generated by cell aggregate containing, instead of the expanded HFM cells, HFM cells harvested from a confluent state.

The applications of the expanded HFM cells produced by the method of the present invention and the cell aggregate containing the expanded HFM cells are not limited to the above-mentioned transplantation to a living body, and the cells and the aggregate are also preferably used in, for example, hair-related research.

In each of the pre-step and main step of the method of the present invention, the HFM cells adhered onto the substrate surface may not be embedded in a hydrogel. In addition, the substrate surface may not be the surface of the hydrogel after gelation. In addition, cells (other cells) except the HFM cells on the substrate surface may not be seeded onto the HFM cells. In addition, the other cells may not be cultured on the HFM cells on the substrate surface. In addition, a layer of the other cells may not be formed on the HFM cells on the substrate surface. In addition, the HFM cells may not be seeded onto the layer of the other cells. In addition, the HFM cells may not be cultured on the layer of the other cells.

Next, specific examples according to this embodiment will be described.

### Example 1

### [Subculture]

The subculture of commercially available human dermal papilla cells (PromoCell GmbH, P2 (number of passages: 2)) was performed. That is, purchased dermal papilla cells (P2) were suspended in a culture solution to prepare a cell suspension. A commercially available dermal papilla cell-growing medium (PromoCell GmbH) was used as the culture solution.

Next, the cell suspension was added, in such an amount that a cell density at the time of the start of culture became about 0.9×10⁴ cells/cm², to a culture dish to seed the dermal papilla cells to the flat bottom surface (area: about 55 cm²) of the culture dish. Then, the two-dimensional culture of the dermal papilla cells (P3) adhered to the bottom surface was performed for 4 days. The culture solution was replaced with a new one once per 2 days.

During the culture for 4 days, the dermal papilla cells proliferated and the cell density reached approximately 70% to approximately 90% of that in a confluent state. A 0.25% trypsin solution was added to the dermal papilla cells before becoming confluent on the 4th day of the culture to detach the dermal papilla cells from the bottom surface of the culture dish. Thus, the dispersed dermal papilla cells were obtained. The dispersed dermal papilla cells were harvested and suspended in a new culture solution to prepare a cell suspension.

Next, the cell suspension was added to a new culture dish, in such an amount that a cell density at the time of the start of culture became about 0.9×10⁴ cells/cm², to seed the dermal papilla cells to the flat bottom surface (area: about 55 cm²) of the culture dish. Then, the two-dimensional culture of the dermal papilla cells (P4) adhered to the bottom surface was performed for 3 days. The culture solution was replaced with a new one once per 2 days.

A 0.25% trypsin solution was added to the dermal papilla cells before becoming confluent on the 3rd day of the culture to detach the dermal papilla cells from the bottom surface of the culture dish, and the dispersed dermal papilla cells were harvested. The harvested dermal papilla cells were suspended in a new dermal papilla cell-growing medium (PromoCell GmbH) to prepare a cell suspension of the dermal papilla cells (P4).

### [Cell Culture]

As the example 1, the long-term adhesion culture of the dermal papilla cells was performed. That is, the cell suspension was added to each flat-bottom well (base area: about 1.9 cm²) of a 24-well plate in such an amount that a cell density at the time of the start of the culture became 1×10⁴ cells/well (about 0.53×10⁴ cells/cm²), to seed the dermal papilla cells to the flat bottom surface of the well. The seeded dermal papilla cells (P5) adhered to the bottom surface of the well. Then, the adhesion culture of the dermal papilla cells was performed for 30 days without passaging the cells. The culture solution was replaced with a new one once per 2 days.

Meanwhile, as the example C1, the long-term suspension culture of the dermal papilla cells was performed. That is, the cell suspension was added to each non-adhesive round-bottom well of a 96-well plate in such an amount that a cell density at the time of the start of the culture became 1×10³ cells/well, to seed the dermal papilla cells into the well. Then, the suspension culture of the dermal papilla cells (P5) was performed for 30 days without passaging the cells. The culture solution was replaced with a new one once per 2 days. In the example C1, the dermal papilla cells aggregated with the lapse of a culture time to form one cell aggregate (spheroid) in each well.

When the diameter of the spheroid is excessively large, the cells in the spheroid undergo necrosis. Accordingly, in the example C1, as described above, the dermal papilla cells were seeded to each well at a cell density of one tenth of that of the example 1 so that a spheroid having such a size that the cells inside did not undergo necrosis was reliably formed.

### [Giemsa Staining]

The Giemsa staining of the dermal papilla cells cultured in the example 1 was performed. Specifically, first, the culture solution was discarded from each well, and the cells adhered to the bottom surface of the well were rinsed with an appropriate amount of methanol. After that, methanol was added to the well, and was held for 10 minutes to fix the cells adhered to the bottom surface of the well. Meanwhile, Giemsa's azur eosin methylene blue solution (1.09204, Merck KGaA) was diluted 200-fold with purified water to prepare a Giemsa staining solution. Then, methanol was discarded from each well, and 1 mL of the Giemsa staining solution was added instead to the well and left to stand for 1 hour. After that, the well was washed well with water for 10 minutes, and was dried for 2 days.

### [Fluorescent Staining]

The fluorescent staining of the dermal papilla cells cultured in the example 1 was performed. Specifically, first, the dermal papilla cells in each well were fixed with 4% paraformaldehyde. Next, 5 minutes of washing the well with PBS was performed twice. Further, a 1% BSA solution was added to the well, and blocking was performed by incubating the mixture in the well for 30 minutes. Then, a solution of a primary antibody (an anti-fibronectin antibody, an anti-type I collagen antibody, or an anti-ALP antibody) diluted with the BSA solution was added to the well, and the mixture in the well was shaken at 4°C overnight. After that, 10 minutes of washing the well with PBS was performed three times.

Next, a solution of a fluorescently labeled secondary antibody was added to the well, and was held for 60 minutes. After that, 10 minutes of washing the well with PBS was performed three times. Further, a DAPI solution was added thereto, and was held for 9 minutes to stain the nuclei of the cells. After that, 5 minutes of washing the well with PBS was performed twice. The dermal papilla cells thus fluorescently stained were observed with a confocal laser microscope.

### [Number of Cells]

The number of the dermal papilla cells cultured in each of the example 1 and the example C1 was determined with a commercially available kit (RealTime-Glo (trademark)). That is, the number of the dermal papilla cells was measured with a kit including a luciferase and a cell-permeable precursor to be converted into a substrate for the luciferase by the reducing abilities of living cells.

### [Expression Amount of Hair Growth-related Gene]

The expression amount of a hair growth-related gene of the dermal papilla cells cultured in each of the example 1 and the example C1 was determined. Specifically, the RNA of the dermal papilla cells was extracted and reverse-transcribed, and then a RT-PCR was performed to determine the expression amount of an alkaline phosphatase (ALP) gene serving as one hair growth-related gene. Primers having the following base sequences were used in the RT-PCR: a forward primer for ALP: 5'-ATTGACCACGGGCACCAT-3'; a reverse primer for ALP: 5'-CTCCACCGCCTCATGCA-3'; a forward primer for GAPDH: 5'-TGGAAATCCCATCACCATCTTC-3'; and a reverse primer for GAPDH: 5'-CGCCCCACTTGATTTTGG-3'.

### [Result]

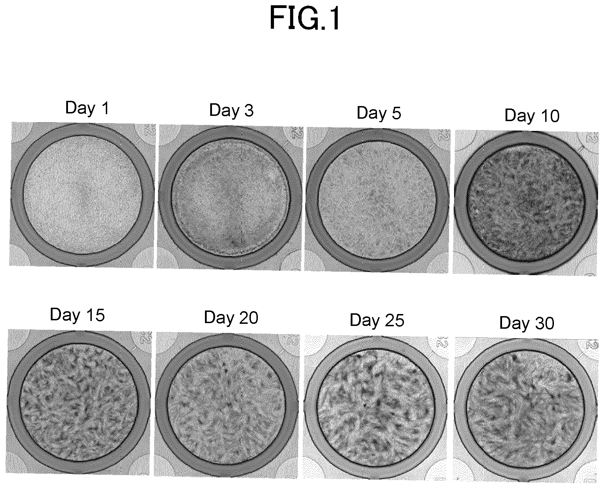

FIG. 1 shows the results of the Giemsa staining of the dermal papilla cells in a well on the 1st day (Day 1), 3rd day (Day 3), 5th day (Day 5), 10th day (Day 10), 15th day (Day 15), 20th day (Day 20), 25th day (Day 25), and 30th day (Day 30) of the culture in the example 1.

As shown in FIG. 1, in the adhesion culture of the example 1, the dermal papilla cells proliferated with the lapse of a culture time, and reached a confluent state on the 5th day (Day 5) of the culture. Herein, in a conventional adhesion culture for expanding the number of dermal papilla cells, the following passage has heretofore been performed: the dermal papilla cells are detached from a substrate surface by enzymatic treatment before the cells reach a confluent state or at the time point when the cells reach the confluent state, followed by harvesting the dispersed dermal papilla cells; and then, the harvested dermal papilla cells are suspended in a new culture solution and seeded onto a new substrate surface.

In contrast, in the example 1, even after the dermal papilla cells had reached the confluent state, their adhesion culture was continued without their passage. As a result, the dermal papilla cells continued to proliferate even after the cells had reached the confluent state. Then, on the 10th day of the culture (Day 10 of FIG. 1), a portion in which the proliferated dermal papilla cells were present at a high density in the state of being stacked (portion densely stained in a photograph corresponding to Day 10 of FIG. 1) was observed in a partial region of the bottom surface of the well.

In the Giemsa staining, the portion in which the dermal papilla cells were stacked was observed as a unique striped pattern in the plan view of the well. Further, the following tendency was observed: a region occupied by the stacked dermal papilla cells in the bottom surface of the well expanded with the lapse of the culture time.

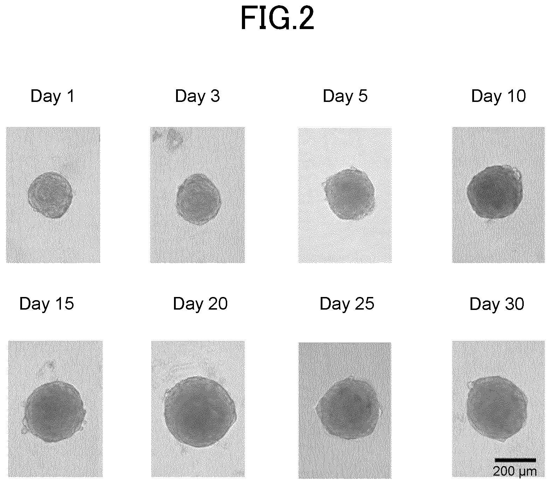

FIG. 2 shows the results of the observation of the dermal papilla cells in a well on the 1st day (Day 1), 3rd day (Day 3), 5th day (Day 5), 10th day (Day 10), 15th day (Day 15), 20th day (Day 20), 25th day (Day 25), and 30th day (Day 30) of the culture in the example C1 with a phase-contrast microscope.

As shown in FIG. 2, in the suspension culture of the example C1, the dermal papilla cells aggregated, and formed a spheroid on the 1st day (Day 1) of the culture. After that, the size of the spheroid increased to some extent, but remained substantially unchanged on and after the 10th day of the culture.

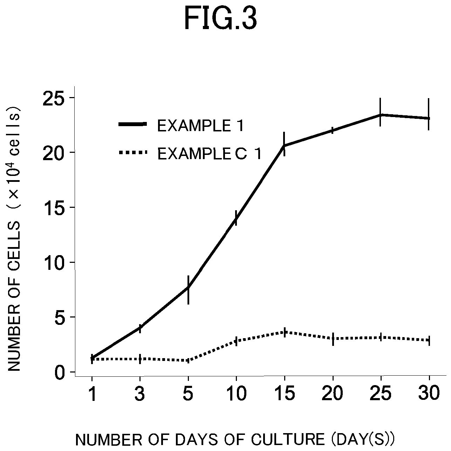

FIG. 3 shows the results of determination of the numbers of the dermal papilla cells in the example 1 (solid line) and the example C1 (dotted line) with time. In FIG. 3, the horizontal axis indicates the number of days of culture (number of days elapsed after the seeding of the dermal papilla cells) (day(s)), and the vertical axis indicates the number of the cells (×10⁴ cells).

As described above, while 1×10⁴ cells were seeded to one well of the 24-well plate in the example 1, 1×10⁴ cells were seeded to 10 wells of the 96-well plate in the example C1. Accordingly, FIG. 3 shows how the 1×10⁴ cells on the 1st day of the culture increased thereafter with the lapse of the culture time. In addition, with regard to the example 1, a value obtained by dividing the number of the cells shown in FIG. 3 by the bottom area of the well (about 1.9 cm²) corresponds to a cell density in the adhesion culture.

As shown in FIG. 3, the proliferation of the dermal papilla cells subjected to the suspension culture to form the spheroid in the example C1 was slight. That is, in the example C1, the number of the dermal papilla cells on the 30th day of the culture was about 2.4 times as large as that on the 1st day of the culture.

In contrast, the dermal papilla cells subjected to the adhesion culture in the example 1 proliferated on the bottom surface of the well, and reached the confluent state on the 5th day of the culture. Specifically, the number of the dermal papilla cells in the example 1 was 1.09×10⁴ cells/well (0.57×10⁴ cells/cm²) on the 1st day of the culture, 3.46×10⁴ cells/well (1.82×10⁴ cells/cm²) on the 3rd day of the culture, and 6.63×10⁴ cells/well (3.49×10⁴ cells/cm²) on the 5th day of the culture.

Further, the dermal papilla cells of the example 1 continued to proliferate even after the cells had reached the confluent state, and their number increased until the 25th day of the culture. Specifically, the number of the dermal papilla cells in the example 1 was 12.02×10⁴ cells/well (6.32×10⁴ cells/cm²) on the 10th day of the culture, 17.76×10⁴ cells/well (9.35×10⁴ cells/cm²) on the 15th day of the culture, 18.97×10⁴ cells/well (9.98×10⁴ cells/cm²) on the 20th day of the culture, 20.18×10⁴ cells/well (10.62×10⁴ cells/cm²) on the 25th day of the culture, and 19.90×10⁴ cells/well (10.48×10⁴ cells/cm²) on the 30th day of the culture. That is, finally, the number of the dermal papilla cells on the 30th day of the culture in the example 1 expanded to about 18.4 times as large as that on the 1st day of the culture, and to about 3.0 times as large as that on the 5th day of the culture.

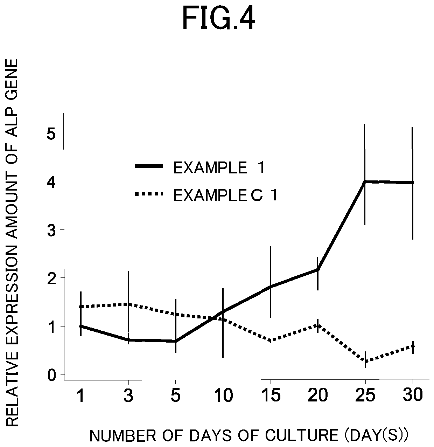

FIG. 4 shows the results of determination of the expression amounts of the ALP gene of the dermal papilla cells cultured in the example 1 (solid line) and the example C1 (dotted line) with time. In FIG. 4, the horizontal axis indicates the number of days of culture (day(s)), and the vertical axis indicates a relative ALP gene expression amount when the expression amount (arithmetic average, n=3) of the ALP gene on the 1st day of the culture in the example 1 is defined as "1".

As shown in FIG. 4, the ALP gene expression amount of the dermal papilla cells of the example C1 (i.e., the spheroid-forming dermal papilla cells) was maintained higher than that of the example 1 until the 5th day of the culture.

In contrast, the ALP gene expression amount of the dermal papilla cells of the example 1 showed a decreasing trend until the 5th day of the culture (i.e., until the cells reached the confluent state), but thereafter turned into an increasing trend, and became comparable to that of the example C1 on the 10th day of the culture.

Specifically, the ALP gene expression amount of the dermal papilla cells of the example 1 reached about 1.3 times as large as that on the 1st day of the culture and about 1.9 times as large as that on the 5th day of the culture on the 10th day of the culture. Further, while the ALP gene expression amount of the dermal papilla cells of the example 1 continued to increase on and after the 10th day of the culture, the ALP gene expression amount of the dermal papilla cells of the example C1 showed a decreasing trend. Then, the ALP gene expression amount of the dermal papilla cells of the example 1 reached about 1.8 times as large as that on the 1st day of the culture and about 2.6 times as large as that on the 5th day of the culture on the 15th day of the culture, reached about 2.2 times as large as that on the 1st day of the culture and about 3.2 times as large as that on the 5th day of the culture on the 20th day of the culture, reached about 4.0 times as large as that on the 1st day of the culture and about 5.8 times as large as that on the 5th day of the culture on the 25th day of the culture, and reached about 3.9 times as large as that on the 1st day of the culture and about 5.8 times as large as that on the 5th day of the culture on the 30th day of the culture. Finally, on the 30th day of the culture, the ALP gene expression amount of the dermal papilla cells of the example 1 reached about 6.9 times as large as that of the example C1.

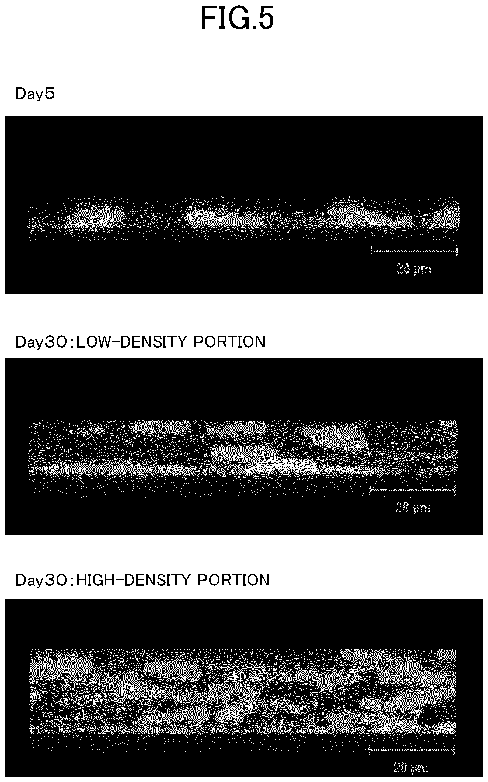

FIG. 5 shows results obtained by: fluorescently staining the cell nuclei of the dermal papilla cells of the example 1 on the 5th day (Day 5) of the culture and the 30th day (Day 30) of the culture with DAPI; and observing a section of a cell layer of the dermal papilla cells with a confocal laser microscope. As described above, on the 5th day (Day 5) of the culture, the dermal papilla cells of the example 1 reached the confluent state, but were adhered to the bottom surface of the well as a single layer without being stacked.

In contrast, on the 30th day (Day 30) of the culture, a portion having a relatively low cell density (Day 30: low-density portion), the portion being stained with a relatively light color by the Giemsa staining, included the dermal papilla cells stacked so as to form about three cell layers, and a portion having a relatively high cell density (Day 30: high-density portion), the portion being stained with a relatively dark color by the Giemsa staining, included the dermal papilla cells stacked so as to form about four to about six cell layers.

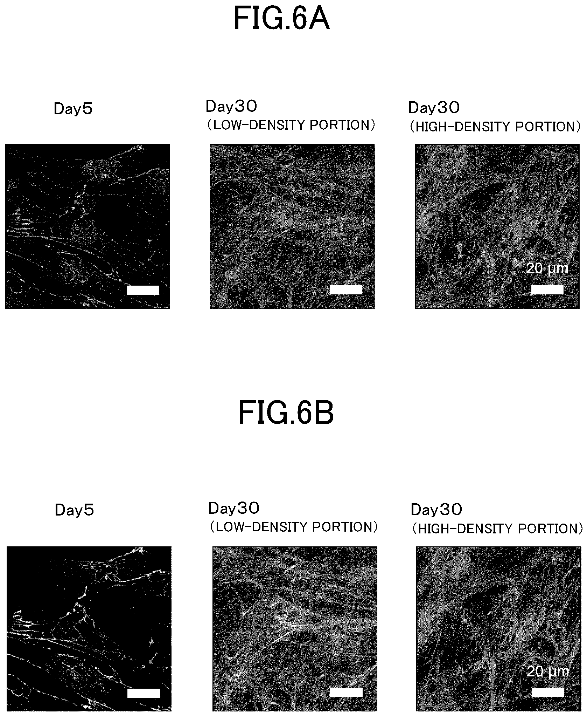

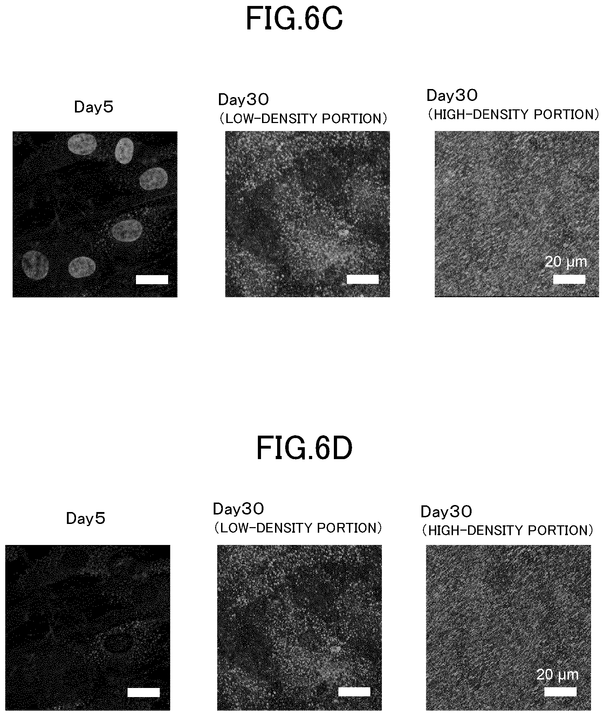

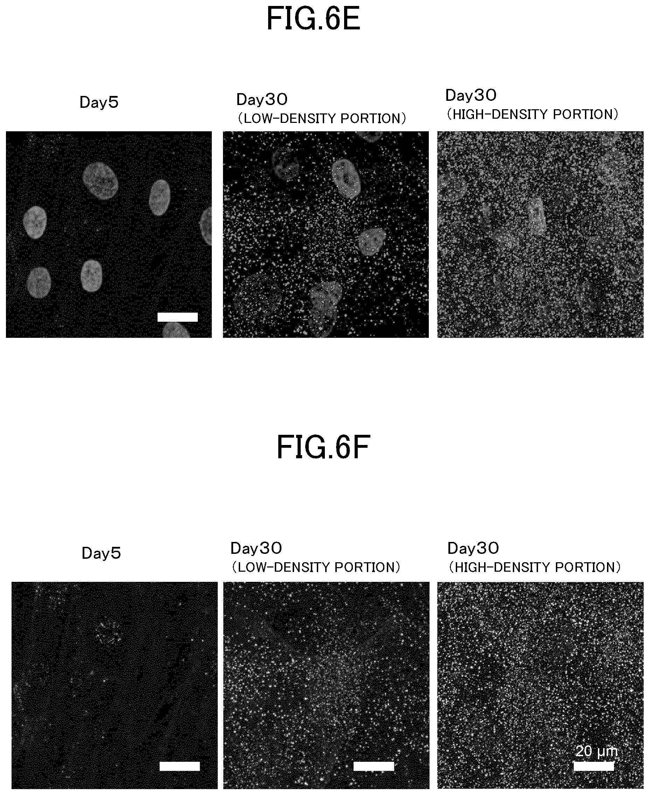

FIG. 6A shows results obtained by: subjecting fibronectin and the cell nuclei in the well of the example 1 on the 5th day (Day 5) of the culture and the 30th day (Day 30) of the culture to immunofluorescent double staining; and observing the stained products with a confocal laser microscope. FIG. 6B shows only the fluorescent staining images of fibronectin among the fluorescent double staining images shown in FIG. 6A. FIG. 6C shows results obtained by: subjecting type I collagen and the cell nuclei in the well of the example 1 on the 5th day (Day 5) of the culture and the 30th day (Day 30) of the culture to immunofluorescent double staining; and observing the stained products with a confocal laser microscope. FIG. 6D shows only the fluorescent staining images of type I collagen among the fluorescent double staining images shown in FIG. 6C. FIG. 6E shows results obtained by: subjecting ALP and the cell nuclei in the well of the example 1 on the 5th day (Day 5) of the culture and the 30th day (Day 30) of the culture to immunofluorescent double staining; and observing the stained products with a confocal laser microscope. FIG. 6F shows only the fluorescent staining images of ALP out of the fluorescent double staining images shown in FIG. 6E.

As shown in FIG. 6A to FIG. 6F, the amounts of fibronectin, type I collagen, and ALP in the well on the 30th day of the culture were significantly larger than those on the 5th day of the culture. That is, it was conceived that with the lapse of the culture time, the dermal papilla cells proliferated, and fibronectin, type I collagen, and ALP produced by the dermal papilla cells accumulated.

In addition, on the 30th day (Day 30) of the culture, larger amounts of fibronectin, type I collagen, and ALP were accumulated in the portion having a relatively high cell density (Day 30: high-density portion) than in the portion having a relatively low cell density (Day 30: low-density portion).

### Example 2

### [Collection of Cells]

Human hair follicle mesenchymal cells were collected from the hair follicles of hairs, which had been collected from an alopecia patient in a medical institution and cold-transported to a laboratory. That is, first, the hair follicles were transferred to a plastic dish, and tissues above dermal papillae out of the hair follicles were cut and separated with a pair of micro-tweezers and a micro-shear blade. Next, the dermal papillae and their surrounding tissues (lower tissues of the hair follicles including the dermal papillae and dermal sheath cups) thus obtained were transferred to a new plastic dish, and were immersed in a solution containing dispase and a collagenase (specifically, an enzyme solution prepared by mixing a solution, which had been obtained by mixing HBSS and PBS at a volume ratio of 1:1, with Dispase II (final concentration: 4.8 U/mL) and Collagenase (final concentration: 100 U/mL), and passing the mixture through a sterilizing filter), followed by enzymatic treatment through incubation at 37°C for 30 minutes.

After that, a supernatant was discarded from the plastic dish, and a new culture solution was added instead thereto, followed by the loosening of the dermal papillae and their surrounding tissues, which had been softened by the enzymatic treatment, through pipetting. As a result, a mixture containing the fragments of the dermal papillae, the surrounding tissues to which part of the dermal papillae adhered, and dispersed cells was obtained. Next, 2 mL of the culture solution containing the mixture was added to each flat-bottom well (base area: about 9.5 cm²) of a 6-well plate, and was cultured for 2 days. During the 2 days of culture, the fragments of the dermal papillae and part of the dispersed cells in the mixture adhered to the bottom surface of the well. On the 2nd day of the culture, the culture solution containing tissue pieces and the cells that were not adhered to the bottom surface of the well was discarded from the well, and a new culture solution was added instead thereto.

Then, the culture of the cells adhered to the bottom surface of the well was continued. The culture solution was replaced with a new one once per 2 days. During the culture period, the cells migrated from the fragments of the dermal papillae adhered to the bottom surface onto the bottom surface. Those hair follicle mesenchymal cells (number of passages: zero: P0) adhered to the bottom surface of the well proliferated on the bottom surface with the lapse of a culture time, and reached a confluent state on the 7th day of the culture.

### [Cell Culture]

A 0.25% trypsin solution was added to the hair follicle mesenchymal cells that had reached the confluent state as described above to detach the cells from the bottom surface of the well. Thus, the hair follicle mesenchymal cells that were dispersed were obtained. The dispersed hair follicle mesenchymal cells were harvested and suspended in a dermal papilla cell-growing medium (PromoCell GmbH) to prepare a cell suspension.

Then, as the example 2, the long-term adhesion culture of the hair follicle mesenchymal cells was performed in the same manner as in the example 1 of Example 1 described above. That is, the cell suspension was added to each flat-bottom well (base area: about 1.9 cm²) of a 24-well plate in such an amount that a cell density at the time of the start of the culture became 1×10⁴ cells/well (about 5.3×10³ cells/cm²), to seed the hair follicle mesenchymal cells to the flat bottom surface of the well. The seeded hair follicle mesenchymal cells (P1) adhered to the bottom surface of the well. Then, the adhesion culture of the hair follicle mesenchymal cells was performed for 30 days without passaging the cells. The culture solution was replaced with a new one once per 2 days.

Meanwhile, as the example C2-1, the long-term suspension culture of the hair follicle mesenchymal cells was performed in the same manner as in the example C1 of Example 1 described above. That is, the cell suspension was added to each non-adhesive round-bottom well of a 96-well plate in such an amount that a cell density at the time of the start of the culture became 1×10³ cells/well, to seed the hair follicle mesenchymal cells into the well. Then, the suspension culture of the hair follicle mesenchymal cells (P1) was performed for 30 days without passaging the cells. The culture solution was replaced with a new one once per 2 days.

In addition, as the example C2-2, the subculture of the hair follicle mesenchymal cells was performed. That is, the cell suspension was added to each flat-bottom well (base area: about 1.9 cm²) of a 24-well plate in such an amount that a cell density at the time of the start of the culture became 1×10⁴ cells/well (about 5.3×10³ cells/cm²), to seed the hair follicle mesenchymal cells to the flat bottom surface of the well. Then, the hair follicle mesenchymal cells (P1) adhered to the bottom surface were cultured for 5 days. The culture solution was replaced with a new one once per 2 days.

After that, in the example C2-2, a 0.25% trypsin solution was added to the hair follicle mesenchymal cells that had reached a confluent state on the 5th day of the culture to detach the cells from the bottom surface of the well, and the hair follicle mesenchymal cells that were dispersed were harvested. The harvested hair follicle mesenchymal cells were suspended in a new culture solution to prepare a cell suspension. Next, the cell suspension was added to each flat-bottom well of a new 24-well plate in such an amount that a cell density at the time of the start of culture became 1×10⁴ cells/well (about 5.3×10³ cells/cm²), to seed the hair follicle mesenchymal cells to the flat bottom surface of the well. Then, the culture of the hair follicle mesenchymal cells (P2) adhered to the bottom surface was performed for 5 days. The culture solution was replaced with a new one once per 2 days.

Further, in the example C2-2, similarly, on the 5th day of the culture of the hair follicle mesenchymal cells (P2) (10th day after the seeding of the hair follicle mesenchymal cells (P1)), the hair follicle mesenchymal cells that had reached a confluent state were harvested and seeded to the bottom surface of a new well. Then, the culture of the hair follicle mesenchymal cells (P3) was performed for 5 days. Further, in the example C2-2, similarly, on the 5th day of the culture of the hair follicle mesenchymal cells (P3) (15th day after the seeding of the hair follicle mesenchymal cells (P1)), the hair follicle mesenchymal cells that had reached a confluent state were harvested and seeded to the bottom surface of a new well. Then, the culture of the hair follicle mesenchymal cells (P4) was performed for 5 days. Further, in the example C2-2, similarly, on the 5th day of the culture of the hair follicle mesenchymal cells (P4) (20th day after the seeding of the hair follicle mesenchymal cells (P1)), the hair follicle mesenchymal cells that had reached a confluent state were harvested and seeded to the bottom surface of a new well. Then, the culture of the hair follicle mesenchymal cells (P5) was performed for 5 days.

### [Giemsa Staining]

The Giemsa staining of the hair follicle mesenchymal cells cultured in the example 2 was performed in the same manner as in Example 1 described above.

### [Number of Cells]

The number of the hair follicle mesenchymal cells cultured in each of the example 2, the example C2-1, and the example C2-2 was determined in the same manner as in Example 1 described above.

### [Expression Amount of Hair Growth-related Gene]

The expression amount of a hair growth-related gene of the hair follicle mesenchymal cells cultured in each of the example 2, the example C2-1, and the example C2-2 was determined in the same manner as in Example 1 described above.

### [Result]

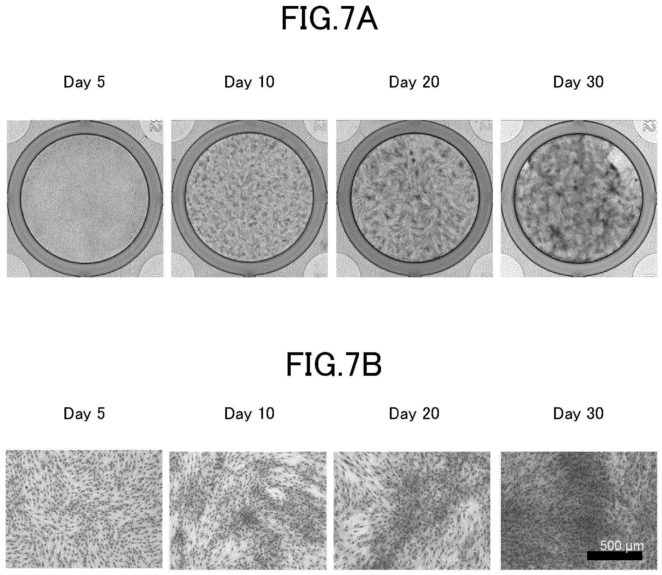

FIG. 7A shows the results of the observation of the Giemsa staining of the hair follicle mesenchymal cells in the well on the 5th day (Day 5), 10th day (Day 10), 20th day (Day 20), and 30th day (Day 30) of the culture in the example 2 at a low magnification. FIG. 7B shows the results of the observation of the Giemsa staining of the hair follicle mesenchymal cells in the well on the 5th day (Day 5), 10th day (Day 10), 20th day (Day 20), and 30th day (Day 30) of the culture in the example 2 at a high magnification.

As shown in FIG. 7A and FIG. 7B, in the adhesion culture of the example 2, the hair follicle mesenchymal cells proliferated with the lapse of the culture time, and reached the confluent state on the 5th day (Day 5) of the culture. In addition, in the example 2, even after the hair follicle mesenchymal cells had reached the confluent state, their adhesion culture was continued without their passage. As a result, the hair follicle mesenchymal cells continued to proliferate even after the cells had reached the confluent state.

Then, on the 10th day of the culture (Day 10 of each of FIG. 7A and FIG. 7B), a portion in which the proliferated hair follicle mesenchymal cells were present at a high density in the state of being stacked (portion densely stained in a photograph corresponding to Day 10 of each of FIG. 7A and FIG. 7B) was observed in a partial region of the bottom surface of the well. Further, the following tendency was observed: the portion in which the hair follicle mesenchymal cells were stacked increased with the lapse of the culture time.

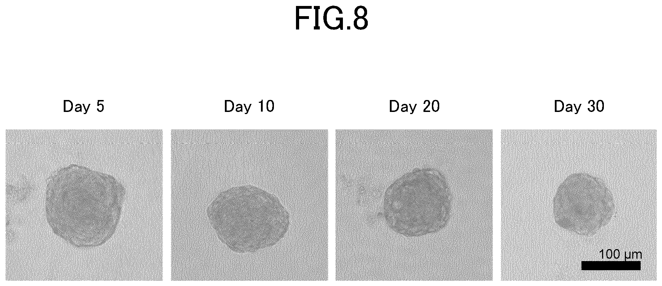

FIG. 8 shows the results of the observation of the hair follicle mesenchymal cells in the well on the 5th day (Day 5), 10th day (Day 10), 20th day (Day 20), and 30th day (Day 30) of the culture in the example C2-1 with a phase-contrast microscope. As shown in FIG. 8, in the suspension culture of the example C2-1, the hair follicle mesenchymal cells aggregated to form a spheroid. No large change in size of the spheroid was observed throughout the culture period.

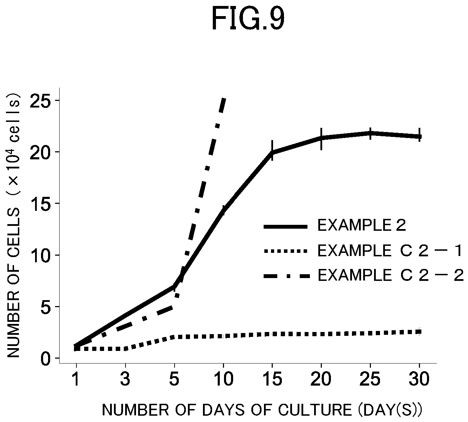

FIG. 9 shows the results of the determination of the numbers of the hair follicle mesenchymal cells in the example 2 (solid line), the example C2-1 (dotted line), and the example C2-2 (dash-dotted line) with time. As in FIG. 3, in FIG. 9, the horizontal axis indicates the number of days of culture, and the vertical axis indicates the number of the cells (×10⁴ cells).

As shown in FIG. 9, the proliferation of the hair follicle mesenchymal cells subjected to the suspension culture to form the spheroid in the example C2-1 was slight. That is, in the example C2-1, the number of the hair follicle mesenchymal cells on the 30th day of the culture was about 2.9 times as large as that on the 1st day of the culture.

In contrast, the hair follicle mesenchymal cells subjected to the adhesion culture in each of the example 2 and the example C2-2 proliferated on the bottom surface of the well, and reached the confluent state on the 5th day of the culture. Specifically, the number of the hair follicle mesenchymal cells in the example 2 was 1.21×10⁴ cells/well (0.64×10⁴ cells/cm²) on the 1st day of the culture, 4.14×10⁴ cells/well (2.18×10⁴ cells/cm²) on the 3rd day of the culture, and 6.91×10⁴ cells/well (3.64×10⁴ cells/cm²) on the 5th day of the culture.

Further, the hair follicle mesenchymal cells of the example 2 continued to proliferate even after the cells had reached the confluent state, and their number increased until the 25th day of the culture. Specifically, the number of the hair follicle mesenchymal cells in the example 2 was 14.28×10⁴ cells/well (7.52×10⁴ cells/cm²) on the 10th day of the culture, 19.90×10⁴ cells/well (10.47×10⁴ cells/cm²) on the 15th day of the culture, 21.34×10⁴ cells/well (11.23×10⁴ cells/cm²) on the 20th day of the culture, 21.82×10⁴ cells/well (11.48×10⁴ cells/cm²) on the 25th day of the culture, and 21.48×10⁴ cells/well (11.30×10⁴ cells/cm²) on the 30th day of the culture. That is, finally, the number of the hair follicle mesenchymal cells on the 30th day of the culture in the example 2 reached about 17.7 times as large as that on the 1st day of the culture, and about 3.1 times as large as that on the 5th day of the culture. In addition, on the 30th day of the culture, the number of the hair follicle mesenchymal cells of the example 2 reached about 8.4 times as large as that of the example C2-1.

Meanwhile, in the example C2-2, as described above, the passage of the hair follicle mesenchymal cells was repeated. As a result, on the 5th day of the culture of the hair follicle mesenchymal cells (P2) (i.e., the 10th day after the seeding of the hair follicle mesenchymal cells (P1)), the number of the hair follicle mesenchymal cells (P2) reached about 21 times as large as that on the 1st day of the culture of the hair follicle mesenchymal cells (P1). In the example C2-2, the number of the hair follicle mesenchymal cells expanded thereafter every time their passage was performed.

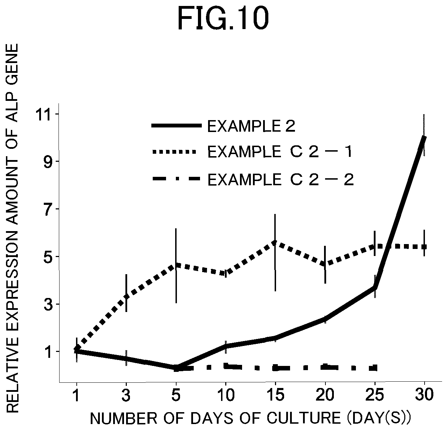

FIG. 10 shows the results of determination of the expression amounts of the ALP gene of the hair follicle mesenchymal cells cultured in the example 2 (solid line), the example C2-1 (dotted line), and the example C2-2 (dash-dotted line) with time. As in FIG. 4, in FIG. 10, the horizontal axis indicates the number of days of culture elapsed after the seeding of the hair follicle mesenchymal cells (P1) (day(s)), and the vertical axis indicates a relative ALP gene expression amount when the expression amount (arithmetic average, n=3) of the ALP gene on the 1st day of the culture in the example 2 is defined as "1".

As shown in FIG. 10, the following tendency was observed: the ALP gene expression amount of the hair follicle mesenchymal cells of the example C2-1 (i.e., the spheroid-forming hair follicle mesenchymal cells) abruptly increased until the 5th day of the culture, and continued to gently increase thereafter.

Meanwhile, the following tendency was observed: the ALP gene expression amount of the hair follicle mesenchymal cells of the example C2-2 reduced with the lapse of the culture time. With regard to the results of the example C2-2 shown in FIG. 10, a value on the 5th day of the culture shows the result of the P1 cells (on the 5th day of the culture of the P1 cells), a value on the 10th day of the culture shows the result of the P2 cells (on the 5th day of the culture of the P2 cells), a value on the 15th day of the culture shows the result of the P3 cells (on the 5th day of the culture of the P3 cells), a value on the 20th day of the culture shows the result of the P4 cells (on the 5th day of the culture of the P4 cells), and a value on the 25th day of the culture shows the result of the P5 cells (on the 5th day of the culture of the P5 cells).

In contrast, the ALP gene expression amount of the hair follicle mesenchymal cells of the example 2 showed a decreasing trend until the 5th day of the culture (i.e., until the cells reached the confluent state), but thereafter turned into an increasing trend, and became larger that of the example 2-1 during a period from the 25th day of the culture to the 30th day of the culture.

Specifically, the ALP gene expression amount of the hair follicle mesenchymal cells of the example 2 reached about 1.2 times as large as that on the 1st day of the culture and about 4.2 times as large as that on the 5th day of the culture on the 10th day of the culture, reached about 1.5 times as large as that on the 1st day of the culture and about 5.4 times as large as that on the 5th day of the culture on the 15th day of the culture, reached about 2.3 times as large as that on the 1st day of the culture and about 8.2 times as large as that on the 5th day of the culture on the 20th day of the culture, reached about 3.7 times as large as that on the 1st day of the culture and about 12.9 times as large as that on the 5th day of the culture on the 25th day of the culture, and reached about 9.9 times as large as that on the 1st day of the culture and about 34.9 times as large as that on the 5th day of the culture on the 30th day of the culture. Finally, on the 30th day of the culture, the ALP gene expression amount of the hair follicle mesenchymal cells of the example 2 reached about 1.9 times as large as that of the example C2-1 and about 34.9 times as large as that of the example C2-2.

### Example 3

### [Cell Culture]

In the example 3, in the same manner as in the example 1 of Example 1 described above, the commercially available human dermal papilla cells were seeded to each flat-bottom well of a 24-well plate, and the adhesion culture of the dermal papilla cells was performed for 30 days without passaging the cells.

In the example C3, in the same manner as in the example C1 of Example 1 described above, the commercially available human dermal papilla cells were seeded to each round-bottom well of a 96-well plate, and the suspension culture of the dermal papilla cells was performed for 30 days without passaging the cells.

### [Transplantation Experiment]

The dermal papilla cells on the 5th day and 30th day of the culture in the example 3 were detached from the bottom surface of the well with a 0.25% trypsin solution, and the dispersed dermal papilla cells were harvested. In addition, a spheroid on each of the 5th day and 30th day of the culture in the example C3 was held in a 0.25% trypsin solution, and then, the dermal papilla cells which form the spheroid were dispersed and harvested through pipetting.

Meanwhile, dorsal skin tissue was collected from a C57BL/6 mouse embryo at an embryonic age of 18 days, and was treated with 48 U/mL dispase at 4°C for 1 hour while being shaken under the condition of from 20 rpm to 30 rpm so that the epithelial layer and mesenchymal layer of the skin tissue were separated. After that, the epithelial layer was treated with a 100 U/mL collagenase for 80 minutes, and was further treated with trypsin for 10 minutes so that their epithelial cells were isolated.

The dermal papilla cells harvested in the example 3 or the dermal papilla cells harvested in the example C3 and the epithelial cells isolated as described above were mixed into a culture solution to prepare a cell suspension containing the dispersed dermal papilla cells and epithelial cells. A mixed medium prepared by mixing a mesenchymal cell culture medium (DMEM+10% FBS+1% penicillin/streptomycin) and HuMedia-KG2 at a volume ratio of 1:1 was used as the culture solution.

Next, the cell suspension was added to each non-adhesive round-bottom well of a 96-well plate in such an amount that the seeding density of the dermal papilla cells became 1×10⁴ cells/well and the seeding density of the epithelial cells became 1×10⁴ cells/well (total number of cells: 2×10⁴ cells/well), to seed the dermal papilla cells and the epithelial cells.

Then, suspension culture serving as the co-culture of the dermal papilla cells and the epithelial cells was performed in an incubator for 3 days. In the co-culture, the dermal papilla cells and the epithelial cells aggregated to form one cell aggregate (hair follicle germ) in each well. That is, with the lapse of a culture time, the cells spontaneously aggregated in each well to form a cell aggregate that was a spherical shape and in a floating state.

The cell aggregates formed in the culture for 3 days as described above were harvested and intradermally transplanted to nude mice. That is, the nude mice were anesthetized through inhalation of isoflurane, and the dorsal part thereof was disinfected with Isodine. Subsequently, a V-lance micro-scalpel (Alcon Japan Ltd.) was used to form incisions for transplantation ranging from the epidermal layer of the skin to a lower part of the dermal layer. Then, the incisions for transplantation were each injected with 30 of the cell aggregates. The care of the nude mice and the transplantation experiment were performed in conformity with the guidelines of the expert committee on animal experiments at Yokohama National University.

### [Result]

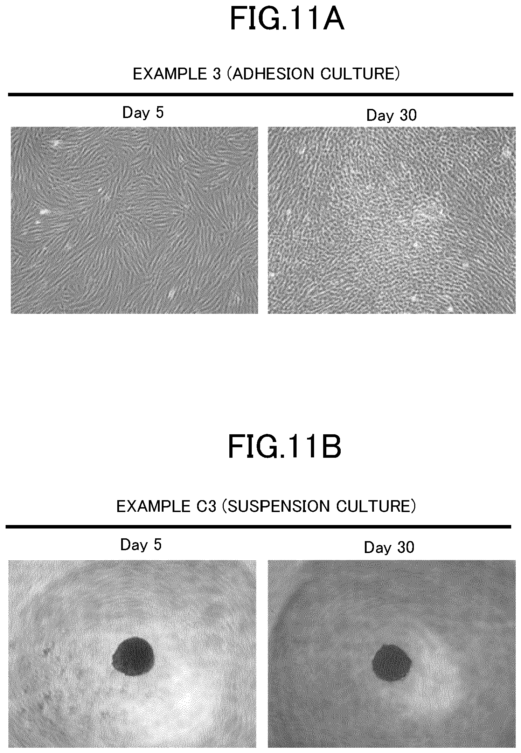

FIG. 11A shows the results of the observation of the dermal papilla cells in the well on the 5th day (Day 5) and 30th day (Day 30) of the culture in the adhesion culture of the example 3 with a phase-contrast microscope. As shown in FIG. 11A, on the 5th day (Day 5) of the culture, the dermal papilla cells reached a confluent state, but the stacking of the dermal papilla cells was not observed, and the dermal papilla cells formed a single layer. Meanwhile, the dermal papilla cells on the 30th day (Day 30) of the culture included a high-density portion in which the dermal papilla cells were stacked in a part in the well.

FIG. 11B shows the results of the observation of the spheroid in the well on the 5th day (Day 5) and 30th day (Day 30) of the culture in the suspension culture of the example C3 with a phase-contrast microscope. As shown in FIG. 11B, no large difference was observed between the appearances of the spheroid on the 5th day (Day 5) and 30th day (Day 30) of the culture.

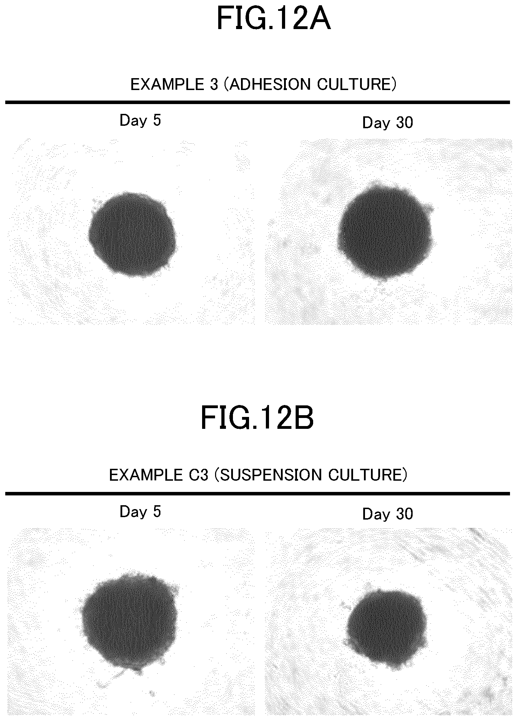

FIG. 12A shows the results of observation of the cell aggregate containing the dermal papilla cells, which were harvested on the 5th day (Day 5) of the adhesion culture in the example 3, and the cell aggregate containing the dermal papilla cells, which have been harvested on the 30th day (Day 30) of the adhesion culture in the example 3, with a phase-contrast microscope. FIG. 12B shows the results of observation of the cell aggregate containing the dermal papilla cells, which were harvested on the 5th day (Day 5) of the suspension culture in the example C3, and the cell aggregate containing the dermal papilla cells, which have been harvested on the 30th day (Day 30) of the suspension culture in the example C3, with a phase-contrast microscope. As shown in FIG. 12A and FIG. 12B, no large difference was observed between the appearances of those cell aggregates.

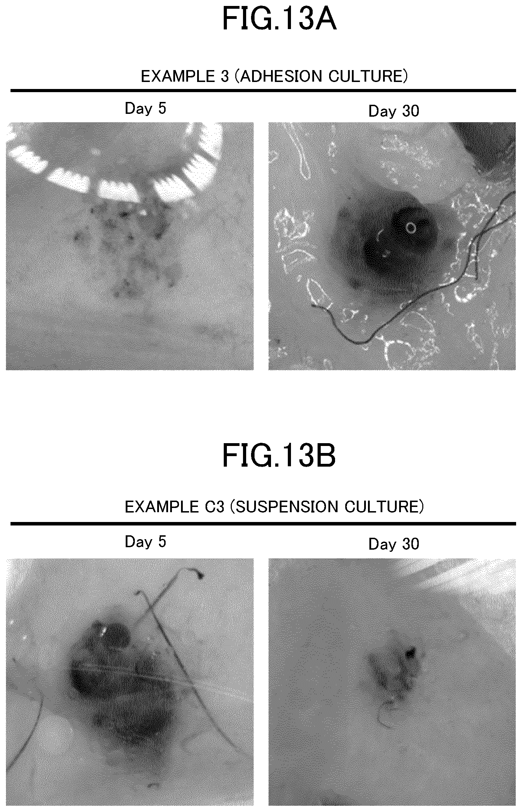

FIG. 13A shows a photograph of the mouse skin 21 days after the transplantation of the cell aggregates containing the dermal papilla cells, which were harvested on the 5th day (Day 5) of the adhesion culture in the example 3, and a photograph of the mouse skin 21 days after the transplantation of the cell aggregates containing the dermal papilla cells, which were harvested on the 30th day (Day 30) of the adhesion culture in the example 3.

As shown in FIG. 13A, the amount of hairs regenerated in the case where the cell aggregates containing the dermal papilla cells, which were harvested on the 30th day (Day 30) of the adhesion culture in the example 3, were transplanted was significantly larger than that in the case where the cell aggregates containing the dermal papilla cells, which were harvested on the 5th day (Day 5) of the adhesion culture therein, were transplanted.

FIG. 13B shows a photograph of the mouse skin 21 days after the transplantation of the cell aggregates containing the dermal papilla cells, which were harvested on the 5th day (Day 5) of the suspension culture in the example C3, and a photograph of the mouse skin 21 days after the transplantation of the cell aggregates containing the dermal papilla cells, which were harvested on the 30th day (Day 30) of the suspension culture in the example C3.

As shown in FIG. 13B, the amount of hairs regenerated in the case where the cell aggregates containing the dermal papilla cells, which were harvested on the 30th day (Day 30) of the suspension culture in the example C3, were transplanted was significantly smaller than that in the case where the cell aggregates containing the dermal papilla cells, which were harvested on the 5th day (Day 5) of the suspension culture in the example C3, were transplanted.

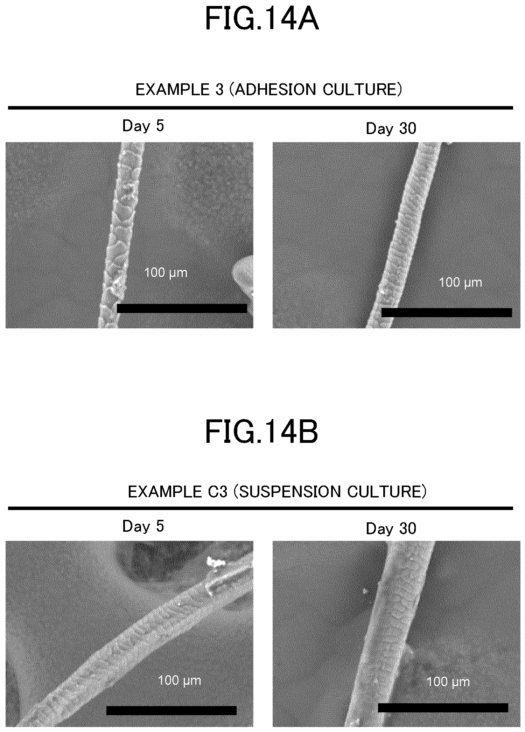

FIG. 14A shows the results of observation of a hair, which grows in a site to which the cell aggregates containing the dermal papilla cells harvested on the 5th day (Day 5) of the adhesion culture in the example 3 were transplanted, and a hair, which grows in a site to which the cell aggregates containing the dermal papilla cells harvested on the 30th day (Day 30) of the adhesion culture therein were transplanted, with a scanning electron microscope (SEM). FIG. 14B shows the results of observation of a hair, which grows in a site to which the cell aggregates containing the dermal papilla cells harvested on the 5th day (Day 5) of the suspension culture in the example C3 were transplanted, and a hair, which grows in a site to which the cell aggregates containing the dermal papilla cells harvested on the 30th day (Day 30) of the suspension culture therein were transplanted, with a SEM. As shown in FIG. 14A and FIG. 14B, it was recognized that the hairs growing in the sites to which the cell aggregates were transplanted each had the same cuticle structure as that of a hair of a living body.

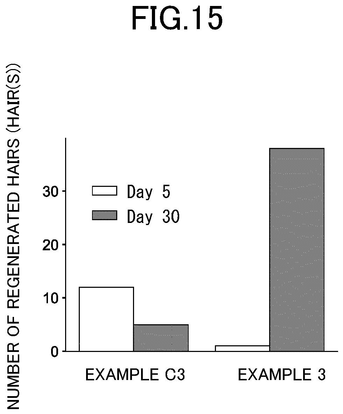

FIG. 15 shows the results of counting the numbers of hairs (vertical axis) regenerated in the mouse skin for four cases shown in FIG. 13A and FIG. 13B. As shown in FIG. 15, the number of hairs regenerated in the case where the cell aggregates containing the dermal papilla cells, which were harvested on the 30th day (Day 30) of the suspension culture in the example C3, were transplanted (Day 30 (blackened bar graph) of EXAMPLE C3) was about 0.42 times as large as that in the case where the cell aggregates containing the dermal papilla cells, which were harvested on the 5th day (Day 5) of the suspension culture in the example C3, were transplanted (Day 5 (outlined bar graph) of EXAMPLE C3).

In contrast, the number of hairs regenerated in the case where the cell aggregates containing the dermal papilla cells, which were harvested on the 30th day (Day 30) of the adhesion culture in the example 3, were transplanted (Day 30 (blackened bar graph) of EXAMPLE 3) was about 38 times as large as that in the case where the cell aggregates containing the dermal papilla cells, which were harvested on the 5th day (Day 5) of the adhesion culture in the example 3, were transplanted (Day 5 (outlined bar graph) of EXAMPLE 3). As described above, it was shown that the dermal papilla cells whose number had been expanded by the long-term and high-density adhesion culture of the example 3 had extremely high hair regeneration abilities.

### Example 4

### [Collection of Cells]

In the same manner as in Example 2 described above, human hair follicle mesenchymal cells were collected from the hair follicles of the hairs of an alopecia patient, and were cultured in each flat-bottom well of a 6-well plate. The hair follicle mesenchymal cells (number of passages: zero: P0) adhered to the bottom surface of the well proliferated on the bottom surface with the lapse of a culture time, and reached a confluent state on the 7th day of the culture.

### [Cell Culture]

In the same manner as in Example 2 described above, the hair follicle mesenchymal cells that had reached the confluent state were detached from the bottom surface of the well, and the dispersed hair follicle mesenchymal cells were suspended in the dermal papilla cell-growing medium to prepare a cell suspension.

Further, as the example 4-1, the long-term adhesion culture of the hair follicle mesenchymal cells was performed in the same manner as in the example 2 of Example 2 described above. That is, the cell suspension was added to each flat-bottom well (base area: about 1.9 cm²) of a 24-well plate in such an amount that a cell density at the time of the start of the culture became 1×10⁴ cells/well (about 5.3×10³ cells/cm²), to seed the hair follicle mesenchymal cells to the flat bottom surface of the well. The seeded hair follicle mesenchymal cells (P1) adhered to the bottom surface of the well. Then, the adhesion culture of the hair follicle mesenchymal cells was performed for 30 days without passaging the cells. The culture solution was replaced with a new one once per 2 days.

In addition, as the example 4-2, the adhesion culture of the hair follicle mesenchymal cells was performed for 30 days in the same manner as in the above-mentioned example 4-1.

In addition, as the example 4C, on the 25th day of the culture in each of the above-mentioned example 4-1 and example 4-2, the hair follicle mesenchymal cells prepared from the hair follicles of the patient were seeded to the flat bottom surface of each well of a 24-well plate in the same manner as in each of the above-mentioned example 4-1 and example 4-2. The seeded hair follicle mesenchymal cells (P1) adhered to the bottom surface of the well. Then, the adhesion culture of the hair follicle mesenchymal cells was performed for 5 days without passaging the cells. The culture solution was replaced with a new one once per 2 days.

### [Harvest of Cultured Cells]

In the example 4-1, on the 30th day of the culture, the hair follicle mesenchymal cells were peeled from the bottom surface of each well with a commercially available cell scraper. As a result, cell layer pieces harvested from the respective wells, the pieces containing the stacked hair follicle mesenchymal cells and being suspended in the medium, were obtained. The cell layer pieces were each a sheet shape immediately after the peeling from the bottom surface, but aggregated during their suspension in the medium to form a cell aggregate.

In the example 4-2, on the 30th day of the culture, a 0.25% trypsin solution was added to each well to peel the hair follicle mesenchymal cells from the bottom surface of each well. As a result, the hair follicle mesenchymal cells harvested from each well and dispersed in the medium were obtained.

In the example 4C, on the 5th day of the culture, that is, the 30th day of the culture in each of the example 4-1 and the example 4-2, a 0.25% trypsin solution was added to each well to peel the hair follicle mesenchymal cells from the bottom surface of each well. As a result, the hair follicle mesenchymal cells harvested from each well and dispersed in the medium were obtained.

### [Transplantation Experiment]

First, epithelial cells were isolated from the skin tissue of a mouse embryo in the same manner as in Example 3 described above. Next, about 2.2×10⁵ epithelial cells and the cell aggregate of the example 4-1 containing about 2.2×10⁵ hair follicle mesenchymal cells, the dispersed hair follicle mesenchymal cells of the example 4-2 whose number was about 2.2×10⁵, or the dispersed hair follicle mesenchymal cells of the example 4C whose number was about 2.2×10⁵, were mixed to prepare three kinds of cell suspensions. Further, each of the cell suspensions was loaded into a microchip having a volume of 200 µL, and a cell aggregate precipitated in the microchip was obtained by subjecting the microchip to centrifugation treatment. Then, each of the cell aggregates was transplanted into the skin of a nude mouse by a patch method in the same manner as in Example 3 described above.

### [Result]

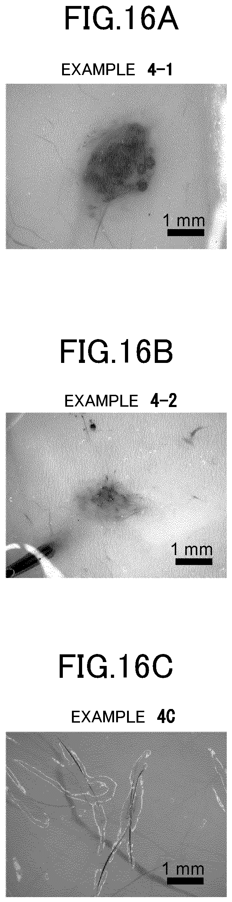

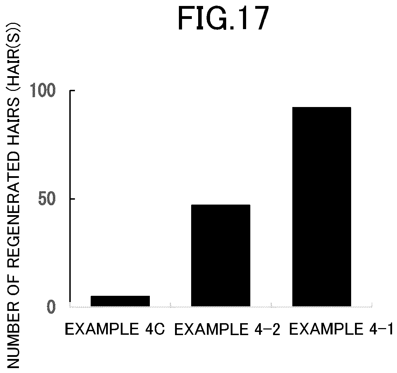

FIG. 16A shows a photograph obtained by photographing a site to which the cells of the example 4-1 were transplanted on the dorsal part of the nude mouse on a 21st day after the transplantation. Similarly, FIG. 16B and FIG. 16C show photographs obtained by photographing a site to which the cells of the example 4-2 were transplanted and a site to which the cells of the example 4C were transplanted on the dorsal parts of the nude mice on the 21st day after the transplantation, respectively. In addition, FIG. 17 shows the result of the counting of the number of hairs growing in each of the site to which the cells of the example 4-1 were transplanted, the site to which the cells of the example 4-2 were transplanted, and the site to which the cells of the example 4C were transplanted, on the 21st day after the transplantation.

As shown in FIG. 16A and FIG. 16B, and FIG. 17, the number of hairs growing in the site to which the cells of the example 4-1 were transplanted, and the number of hairs growing in the site to which the cells of the example 4-2 were transplanted, were significantly larger than that in the example 4C. Further, the number of hairs growing in the site to which the cells of the example 4-1 were transplanted was significantly larger than that in the example 4-2.

### Example 5

### [Cell Culture]

The commercially available human dermal papilla cells were subcultured in the same manner as in the example 1 of Example 1 described above to provide dermal papilla cells whose number of passages was 6. Then, as example 5, the dermal papilla cells (P6) were seeded to each flat-bottom well of a 24-well plate, and the adhesion culture of the dermal papilla cells was performed for 55 days without passaging the cells.

### [Harvest of Cultured Cells]

In the example 5, in the same manner as in the example 4-1 of Example 4 described above, on the 55th day of the culture, the dermal papilla cells were peeled from the bottom surface of each well with a commercially available cell scraper, and a cell aggregate suspended in the medium was harvested. Next, the harvested cell aggregate was stained with a commercially available reagent for fluorescent staining (DiD Cell-Labeling Solution). In addition, as the example 5C, the dispersed dermal papilla cells (P6) that had not been subjected to any long-term adhesion culture were stained with the commercially available reagent for fluorescent staining (DiD Cell-Labeling Solution).

### [Transplantation Experiment]

In the example 5, the stained cell aggregate was subcutaneously transplanted to three sites separated from each other in the dorsal portion of each of two SHO mice (5 weeks old, The Jackson Laboratory). In addition, in the example 5C, the stained dermal papilla cells were subcutaneously transplanted in a dispersed state to three sites separated from each other in the dorsal portion of each SHO mouse.

### [Measurement of Fluorescence Intensity]

Fluorescence intensities in the respective transplanted sites of the mice were measured with a commercially available in vivo fluorescent imaging apparatus (IVIS (trademark) Lumina Series III, PerkinElmer, Inc.) immediately after the transplantation and on the 7th day after the transplantation. Then, each of the transplanted sites was evaluated for relative fluorescence intensity, which was the ratio of the fluorescence intensity on the 7th day after the transplantation to the fluorescence intensity immediately after the transplantation. The fluorescence intensities were measured with commercially available software (Living Image (trademark) Software, PerkinElmer, Inc.).

### [Result]

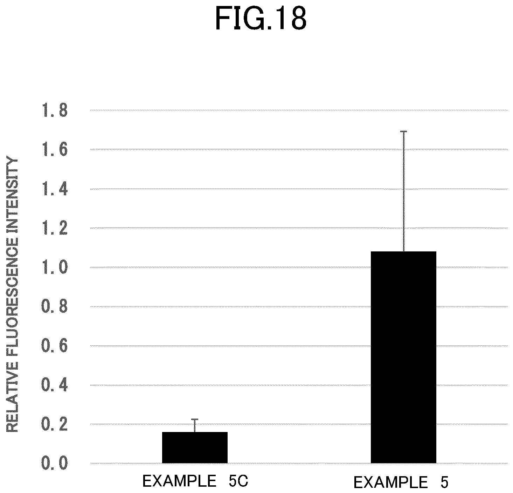

FIG. 18 shows the result of evaluation of the relative fluorescence intensity in each of the sites to which the cells of the example 5 were transplanted, and the sites to which the cells of the example 5C were transplanted. As shown in FIG. 18, the relative fluorescence intensity in each of the sites to which the cells of the example 5 were transplanted was significantly larger than that in the example 5C.

## Claims

1. A method of producing expanded hair follicle mesenchymal cells, comprising a main step of culturing hair follicle mesenchymal cells adhered to a substrate surface from a confluent state without passaging the cells,
wherein the main step includes culturing the hair follicle mesenchymal cells to make the cells proliferate further from the confluent state until the proliferated hair follicle mesenchymal cells are stacked to form cell layers including two or more stacked layers in at least part of the substrate surface.

2. The method of producing expanded hair follicle mesenchymal cells according to claim 1, wherein the main step includes culturing the hair follicle mesenchymal cells until a time period of 120 hours or more elapses from the confluent state.

3. The method of producing expanded hair follicle mesenchymal cells according to claim 1, wherein the main step includes culturing the hair follicle mesenchymal cells until a cell density of the hair follicle mesenchymal cells on the substrate surface reaches 7×10⁴ cells/cm² or more.

4. The method of producing expanded hair follicle mesenchymal cells according to claim 1, wherein the main step includes culturing the hair follicle mesenchymal cells for 50 hours or more in a state where a cell density of the hair follicle mesenchymal cells on the substrate surface is 7×10⁴ cells/cm² or more.

5. The method of producing expanded hair follicle mesenchymal cells according to claim 1, wherein the main step includes culturing the hair follicle mesenchymal cells until a cell density of the hair follicle mesenchymal cells on the substrate surface reaches 1.5 or more times as high as that in the confluent state.

6. The method of producing expanded hair follicle mesenchymal cells according to claim 1, wherein the main step includes culturing the hair follicle mesenchymal cells until an expression amount of each of one or more hair growth-related genes of the hair follicle mesenchymal cells increases to 1.5 or more times as large as that in the confluent state.

7. The method of producing expanded hair follicle mesenchymal cells according to claim 1, further comprising a pre-step to be performed prior to the main step,
wherein the pre-step includes culturing the hair follicle mesenchymal cells that have not yet reached the confluent state on the substrate surface to make the cells proliferate until the hair follicle mesenchymal cells reach the confluent state.

8. The method of producing expanded hair follicle mesenchymal cells according to claim 7, wherein the pre-step includes culturing the hair follicle mesenchymal cells at a cell density of 2.5×10⁴ cells/cm² or less to make the cells proliferate until the hair follicle mesenchymal cells reach the confluent state.

9. The method of producing expanded hair follicle mesenchymal cells according to claim 7, wherein the main step includes culturing the hair follicle mesenchymal cells until a time period of 200 hours or more elapses from a start of the culture of the hair follicle mesenchymal cells in the pre-step.

10. The method of producing expanded hair follicle mesenchymal cells according to claim 7, wherein the main step includes culturing the hair follicle mesenchymal cells until an expression amount of each of one or more hair growth-related genes of the hair follicle mesenchymal cells increases to 1.1 or more times as large as that at a time point when 24 hours elapses from a start of the culture of the hair follicle mesenchymal cells in the pre-step.

11. The method of producing expanded hair follicle mesenchymal cells according to claim 1, further comprising a harvesting step of detaching the hair follicle mesenchymal cells, which have been obtained through the culture on the substrate surface from the confluent state without the passage in the main step, from the substrate surface, followed by harvesting the detached cells.

12. The method of producing expanded hair follicle mesenchymal cells according to claim 1, wherein the hair follicle mesenchymal cells are one or more selected from the group consisting of: dermal papilla cells; and dermal sheath cup cells.

13. A method of producing a cell aggregate, comprising forming a cell aggregate containing expanded hair follicle mesenchymal cells produced by the method of any one of claims 1 to 12.

14. A method of producing a composition for transplantation, comprising preparing a composition for transplantation containing expanded hair follicle mesenchymal cells produced by the method of any one of claims 1 to 12.

15. A hair regeneration method, comprising transplanting, to a living body, expanded hair follicle mesenchymal cells produced by the method of any one of claims 1 to 12.
